(19) Europäisches Patentamt
European Patent Office
Office européen des brevets

(11) Publication number 0 004 773
B1

(12) # EUROPEAN PATENT SPECIFICATION

(45) Date of publication of patent specification: 29.04.81

(51) Int. Cl.³: C 07 J 31/00, C 07 J 71/00, A 61 K 31/56, A 61 K 31/58 // C07J3/00, C07J5/00

(21) Application number: 79300549.7

(22) Date of filing: 03.04.79

(54) 17-Beta-thiocarboxylic acid esters of 4-halo-3-oxoandrost-4-enes, their pharmaceutical use and processes for their preparation.

(30) Priority: 05.04.78 US 893390

(43) Date of publication of application: 17.10.79 Bulletin 79/21

(45) Publication of the grant of the patent: 29.04.81 Bulletin 81/17

(84) Designated Contracting States: BE CH DE FR GB NL SE

(56) References cited: US-A-3 989 686

(73) Proprietor: SYNTEX (U.S.A.) INC., 3401 Hillview Avenue, Palo Alto, California 94304 (US)

(72) Inventor: Alvarez, Francisco S., 1089 Rochester Court, Sunnyvale California 94087 (US)

(74) Representative: Armitage, Ian Michael et al, MEWBURN ELLIS & CO. 70/72 Chancery Lane, London WC2A 1AD (GB)

EP 0 004 773 B1

Note Within nine months from the publication of the mention of the grant of the European patent any person may give notice to the European Patent Office of opposition to the European patent granted. Notice of opposition shall be filed in a written reasoned statement. It shall not be deemed to have been filed until the opposition fee has been paid (Art. 99(1) European patent convention).

BUNDESDRUCKEREI BERLIN

17β-thiocarboxylic acid esters of 4-halo-3-oxoandrost-4-enes, their pharmaceutical use and processes for their preparation

This invention relates to 4-halo-3-oxoandrost-4-ene 17β-thiocarboxylates and to pharmaceutical antiinflammatory compositions comprising such compounds. The invention further relates to processes for the preparation of these novel compounds.

Certain 3-oxoandrost-4-ene 17β-carboxylic acids which are substituted at the 9 position with chlorine or fluorine and at the 11 position with oxo or hydroxy or chloro groups are known. See for example U.S. 3,828,080. It is known that 3-oxoandrost-4-ene 17β-carboxylic acids may be substituted at both the 9α and 6α positions with fluoro. See for example U.S. 3,636,010 and U.S. 4,093,721.

It is also known from U.S. 3,989,686 to Phillipps et al of Glaxo that steroids of Formula (II)

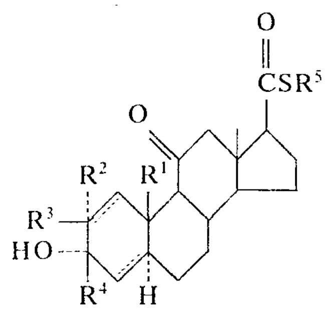

(II)

wherein

- $R^1$ is H or $CH_3$;
- $R^2$ is H or $CH_3$;
- $R^3$ is H or, when $R^2$ is H, $C_{1-6}$ alkoxy, $C_{1-5}$ alkyl, thiocyanato or halogen;
- $R^4$ is H or $CH_3$;
- $R^5$ is $C_{1-6}$ alkyl optionally substituted by halo or $NR^6R^7$, where $R^6$ and $R^7$ arethe same or different, $C_{1-6}$ alkyl or $R^6$ and $R^7$ together with N are morpholino, thiamorpholine or morpholino substituted with $C_{1-6}$ alkyl; and

the dotted lines in the »A« ring represent an optional double bond at these positions;
are useful as anaesthetics.

Methyl 3β-acetoxyallothiol-cholonate and methyl 3β-acetoxy-etiothiochol-5-enate are also known compounds. See, e.g., Jerger at al, Helv. Chem. Acta. 29, 684–92 (1946).

A heretofore unknown series of 3-oxoandrost-4-ene 17β-thiocarboxylates being substituted at the 4 position with fluoro, chloro or bromo and optionally substituted at the 6 position with fluoro or chloro has been discovered and is disclosed herein. The compounds exhibit good anti-inflammatory activity and few adverse side effects.

One aspect of this invention is a compound chosen from those represented by Formula (I)

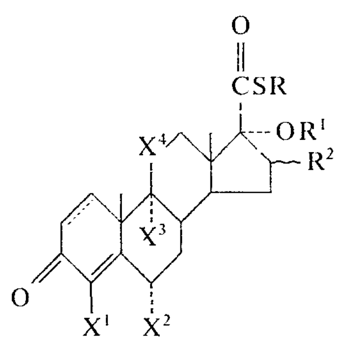

(I)

wherein

- $X^1$ is fluoro, chloro or bromo;
- $X^2$ is fluoro, chloro or hydrogen;
- $X^3$ is fluoro, chloro, bromo or hydrogen;
- $X^4$ is $=C=O$ or

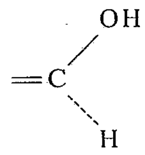

or may also be

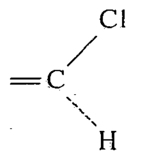

when $X^3$ is chloro;

R is alkyl of 1 to 6 carbon atoms, or phenyl or benzyl optionally substituted with one substituent chosen from alkyl of 1 to 4 carbon atoms, alkoxy of 1 to 4 carbon atoms and halo;

$R^1$ is hydrogen or alkanoyl of 2 to 6 carbon atoms when $R^2$ is hydrogen, $\alpha$-methyl or $\beta$-methyl, or $OR^1$ and $R^2$ together represent 16$\alpha$,17$\alpha$-isopropylidenedioxy; and

the solid and broken lines between C-1 and C-2 represent a single or double bond.

Another aspect of this invention is an antiinflammatory pharmaceutical composition which comprises at least one suitable pharmaceutical excipient in combination with a therapeutically effective amount of a compound chosen from those represented by Formula (I), as defined above, wherein each of the substituents are as defined. Particularly valuable compounds for this composition are set forth hereafter.

Still another aspect of this invention is, for use in treating an inflamed condition in a mammal, a compound of Formula (I), above, wherein substituents are as defined above.

Still another aspect of this invention is a process for preparing a compound of this invention, and is discussed hereafter.

One subgroup of the broad aspect of the invention comprises those compounds represented by Formula (I) wherein $X^1$ is fluoro or chloro; $X^2$ is fluoro or hydrogen; $X^3$ is fluoro, chloro or hydrogen; $X^4$ is

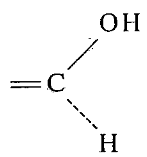

or may also be

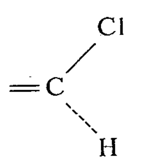

when $X^3$ is chloro; R is alkyl of 1 − 6 carbon atoms; and $R^1$ is alkanoyl of 2 − 6 carbon atoms when $R^2$ is $\alpha$-methyl or $\beta$-methyl or $OR^1$ and $R^2$ together are isopropylidenedioxy. A subdivision of this subgroup includes those compounds of Formula (I) wherein $R^2$ is $\alpha$-methyl or $\beta$-methyl, $X^1$ is fluoro, $X^2$ is hydrogen or fluoro, $X^3$ is hydrogen or fluoro, $X^4$ is

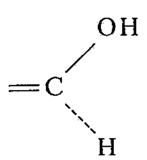

and there is a double bond between C-1 and C-2. Of the compounds of this subdivision, the preferred compounds are represented by Formula (I) wherein $X^1$, $X^2$ and $X^3$ are all fluoro, R is methyl and $OR^1$ is acetate or propionate.

Another subgroup of the compounds of this invention includes those compounds represented by Formula (I) wherein R is alkyl of 1–6 carbon atoms, $OR^1$ and $R^2$ together represent 16α,17α-isopropylidenedioxy; $X^4$ is

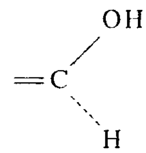

$X^1$ is fluoro and $X^2$ and $X^3$ are independently hydrogen or fluoro. A subdivision of this subgrounp includes those compounds of Formula (I) where R is methyl or ethyl (particularly methyl). Of the compounds of this subdivision, the preferred compounds are represented by Formula (I) wherein $X^1$, $X^2$ and $X^3$ are all fluoro.

In defining the compounds of this invention the term »alkyl« includes both straight chain and branched alkyl groups, thus alkyl of 1–6 carbon atoms includes methyl, ethyl, n-propyl, isopropyl, n-butyl, isobutyl, t-butyl, pentyl. isoamyl, n-hexyl »and the like«. The phenyl and benzyl substituents may be substituted on the phenyl ring at the 2, 3 or 4 positions with one substituent such as alkoxy of 1–4 carbons (e.g. methoxy, ethoxy, n-propoxy, t-butoxy and the like), alkyl of 1–4 carbons (e.g., methyl, ethyl, isopropyl, n-propyl, t-butyl, n-butyl, etc.), or a halo such as fluoro, chloro, bromo or iodo. Preferably the substitution is at the 2 or 4 positions.

The term »alkanoyl« refers to a radical of the formula

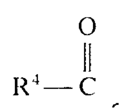

wherein $R^4$ is alkyl of 1–5 carbon atoms and includes e.g. acetyl, propionyl, butyryl, valeryl, caproyl, »and the like«.

In naming the compound of this invention the substituents present on the androstane ring shall be included alphabetically and the compounds shall be alkyl (or phenyl or benzyl) 17β-thiocarboxylates. For example, if in Formula (I), above, $X^1$ and $X^2$ are fluoro, $X^3$ and $X^4$ are chloro, R is methyl, $R^1$ is acetoxy and $R^2$ is α-methyl the name is methyl 17α-acetoxy-9α,11β-dichloro-4,6α-difluoro-16α-methyl-3-oxo-androsta-1,4-diene-17β-thiocarboxylate. If, on the other hand, R is hydrogen but $X^1$, $X^2$, $X^3$, $X^4$, $R^1$ and $R^2$ are the same the compounu is named 17α-acetoxy-9α,11β-dichloro-4,6α-difluoro-16α-methyl-3-oxoandrosta-1,4-diene 17β-thiocarboxylic acid.

Another aspect of this invention is the preparation of compounds of the invention, for example prepared by converting an appropriate androst-4-ene 17β-carboxylic acid (or a reactive derivative thereof) to the corresponding 17β-thiocarboxylate of this invention. This conversion is carried out by reacting an appropriate androst-4-ene 17β-carboxylic acid (or a reactive derivative thereof) with a molar excess (about 1.05 to about 5 molar equivalents based on the steroid) of a suitable basic salt, e.g. an alkali metal salt, of a compound of the formula RSH where R is the appropriate alkyl, benzyl or phenyl moiety. Representative alkali metal salts include sodium methyl sulfide, sodium ethyl sulfide, sodium benzyl sulfide, sodium phenyl sulfide, potassium methyl sulfide, and the like. The alkali metal salt can be reacted directly with the reactive derivative of the 17β-carboxylic acid, or the salt can be formed in situ by mixing an alkali metal hydride, such as sodium hydride or potassium hydride, with an alkyl, phenyl or benzyl sulfide. The thioesterification reaction readily takes place at temperature of about 10° to 100° C (preferably at ambient temperature of about 20–25° C) in a suitable inert solvent such as dimethylformamide, diethylformamide, dimethylacetamide, and the like.

The reactive derivative of the 17β-carboxylic acid may be an acid chloride, but is preferably a mixed anhydride, such as the dialkyl phosphate ester prepared by reacting a dialkyl (1–4 carbons) chlorophosphate (e.g. diethyl chlorophosphate) with the appropriate 17β-carboxylic acid in an inert solvent such as tetrahydrofuran (THF) under an inert atmosphere (nitrogen) at temperatures of about 10–50° C, preferably about 20–25° C.

Several overall processes may be employed to prepare the compounds of this invention from known pregnanes. These are outlined in Reaction Sequences A–C.

Reaction Sequence A

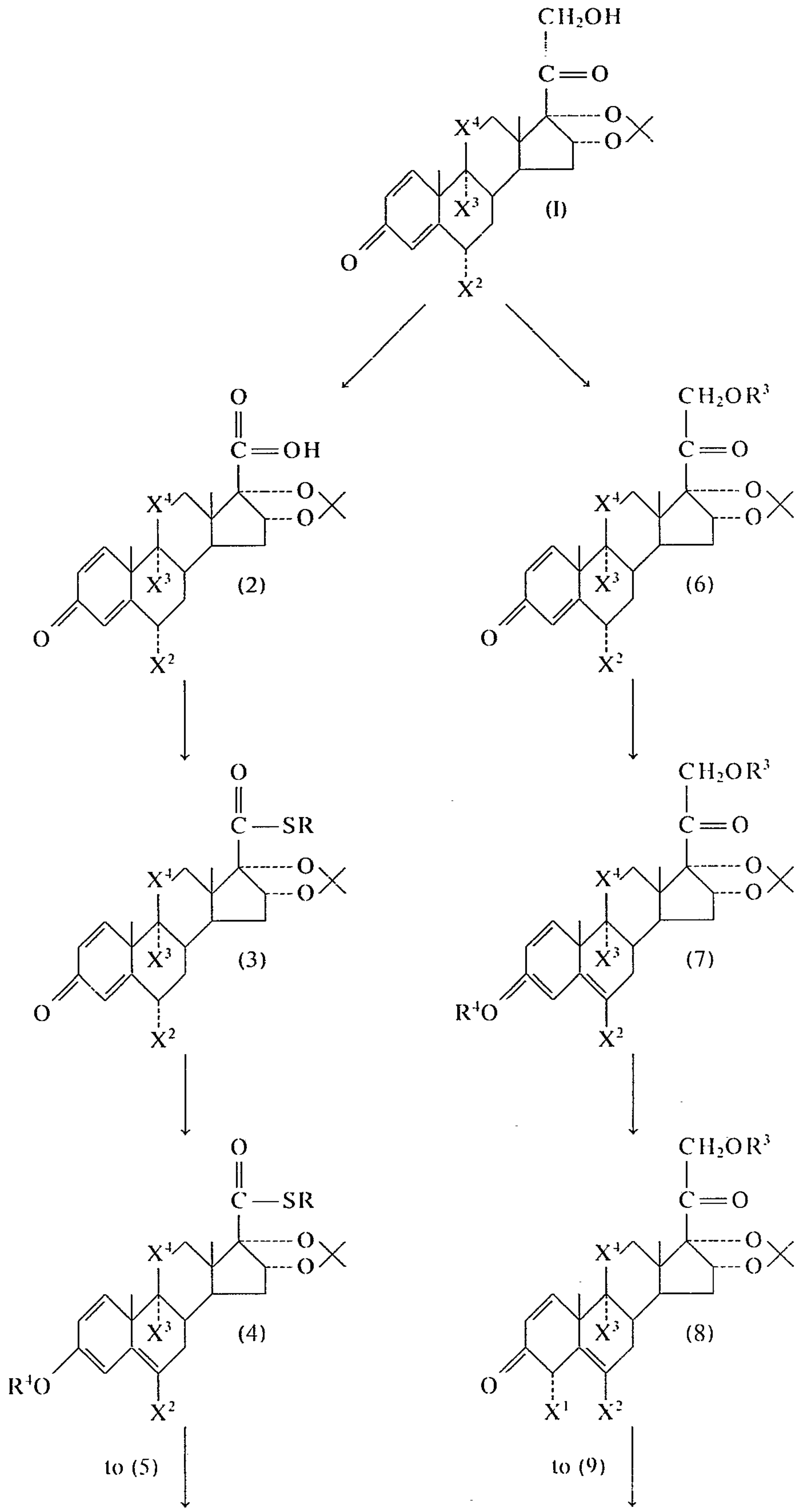

(I)

(2)

(6)

(3)

(7)

(4)

(8)

to (5)

to (9)

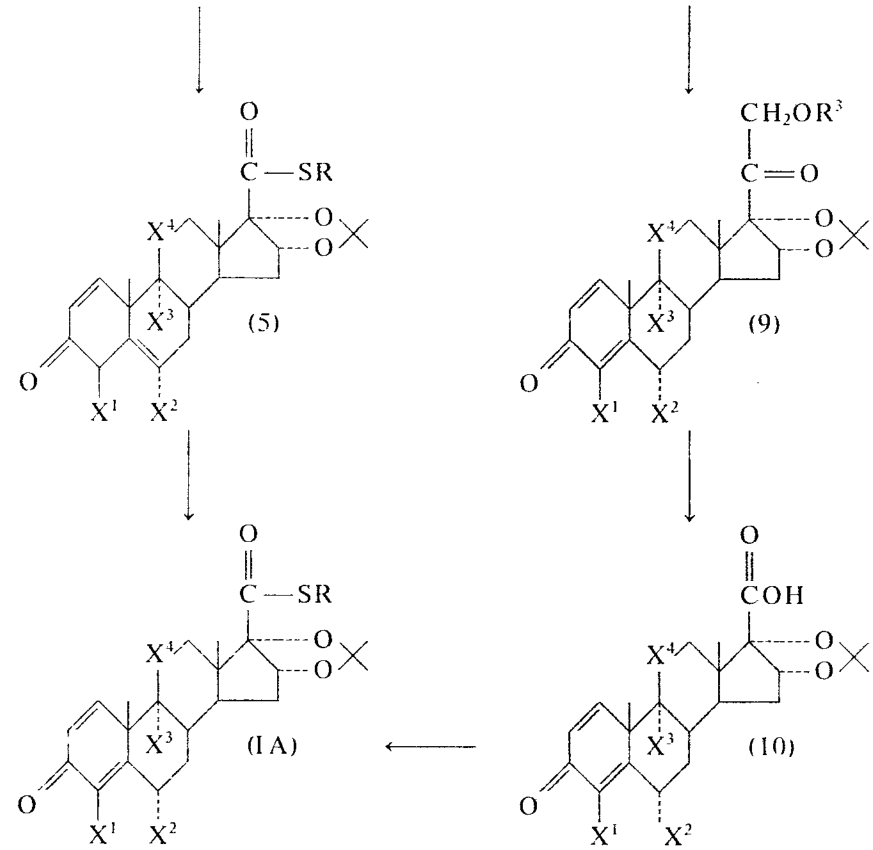

(5) (9) (IA) (10)

Reaction Sequence (A) sets forth essentially a three-part process for the preparation of the 16α,17α-acetonide compounds of this invention, the parts of which may be carried out in any order. One part is to eliminate the 21 carbon atom from a suitable 21-hydroxy 6α-fluoro pregnane, or a suitable ester thereof; another part is to fluorinate, chlorinate or brominate at the 4-position, and the third part is to form the thiocarboxylate. In Reaction Sequence (A), R is a suitable alkyl, benzyl or phenyl group as defined in the broadest aspect of the invention; $R^3$ is alkanoyl of 2–6 carbons; $X^4$ is

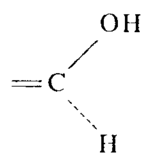

or

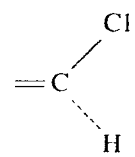

$R^4$ is methyl or ethyl; and $X^3$ is fluoro, chloro or bromo, and $X^1$ and $X^2$ are as hereinbefore defined. In the Reaction Sequence, the fluorination (chlorination or bromination) is a threestep process, the elimination of the 21-carbon atom is essentially a one-step process, and the thioesterification is a one-step process.

The elimination of a 21 carbon atom from a suitable pregnane, represented by Formulas (1) or (9), is readily accomplished by any means known in the art such as using sodium hypobromite or hypoiodite as taught in U.S. 2,769,822 or by using sodium periodate. Preferably, however the elimination of the 21-carbon atom is carried out by using an alkali metal carbonate in alcohol in the presence of oxygen. In the latter case the reaction is carried out at room temperature and atmospheric pressure while the source of oxygen is preferably air. Generally the reaction will be completed within less than 72 hours with a constant stream of air being bubbled into a stirred reaction mixture to give a compound of Formula (2) or (10), respectively.

Once a compound represented by formula (2) or (10) is obtained, it is readily thioesterified by methods discussed hereinbefore to give the 17β-thiocarboxylate represented by formula (3) or (IA), respectively.

The compound represented by formula (3), in turn, is fluorinated, chlorinated or brominated (collectively referred to as halogenated) at the 4-position using the three step halogenation technique.

The first step of the halogenation process is performed by reacting a compound of formula (3) to form a compound of formula (4) wherein $R^4O$ is methoxy or ethoxy. This is carried out by reacting, for example, a large molar excess of trimethyl orthoformate in methanol or triethyl orthoformate in ethanol in the presence of a catalytic amount (i.e. less than 5% by weight) of a suitable acid catalyst

such as fuming sulfuric acid at reflux temperature or less. About 50—55° C is preferred. Generally the molar ratio of the orthoformate to steroid is about 10 : 1 to about 30 : 1. Once the reaction is complete a base is added to neutralize the acid and the resulting product represented by formula (4) is recovered and purified using methods well known in the art such as recrystallization, chromatography, etc.

The compound represented by formula (4) is then halogenated using perchloryl fluoride ($ClO_3F$) or trifluoromethoxy fluoride ($CF_3OF$) as a fluorinating agent, a source of positive chlorine such as N-chlorosuccinimide, dichlorohydantoin, etc. as a chlorinating agent, or N-bromosuccinimide as a brominating agent to form the 3-keto-4α-fluoro (chloro or bromo) steroid represented by formula (5).

In the case of $ClO_3F$, which is a gas, an approximately equimolar amount, i.e. about 1 to 1.1 moles $ClO_3F$ per mole compound of formula (4) is metered into a mixture of the compound in a solution which is a major amount of acetone, preferably 90% by volume, and a minor amount water, preferably about 10%, over a period of about 1—3 hours at about —75° to 20° C, preferably starting at about —75° C and allowing the reaction mixture to slowly warm to ambient temperatures. Dichlorohydantoin or N-bromosuccinimide are reacted using a solvent such as acetone and water or tetrahydrofuran and water to dissolve the reactants and adding the halogenating solution to the compound in a similar solvent at about —50° C to about 50° C.

In another aspect of this invention, the resulting compound of formula (5), in turn, is recovered and reacted with a suitable base such as an alkali metal carbonate, e.g. potassium carbonate, in a suitable oxygenated hydrocarbon solvent such as an alkanol, e.g. methanol, in an inert atmosphere, to rearrange the pregna-1,5-diene to form the desired 4-fluoro(4-chloro or 4-bromo)-3-oxopregna-1,4-diene represented by formula (IA).

It will be appreciated that a compound represented by formula (7) is readily halogenated to a compound of formula (9) through intermediates (7) and (8) by the same halogenation process as discussed above.

Once the desired 3-keto-androsta-1,4-diene represented by formula (IA) is obtained, the compound may be readily selectively hydrogenated across the 1—2 bond by any of the means known in the art to obtain the corresponding 3-keto-androst-4-ene.

Reaction Sequence B

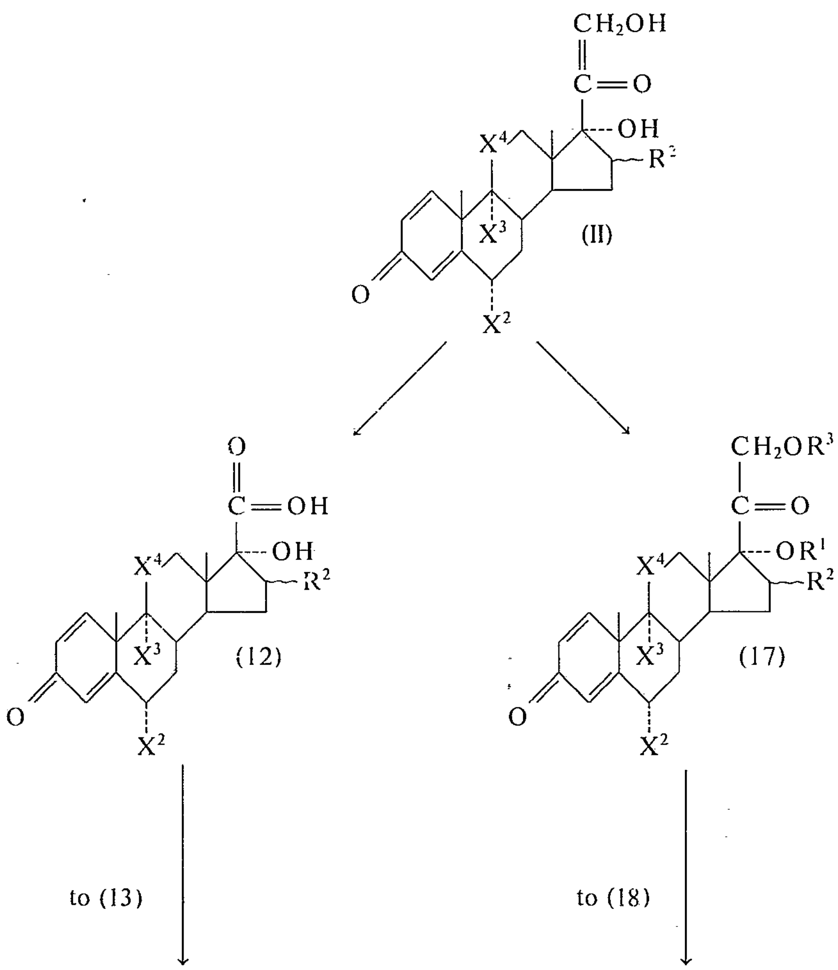

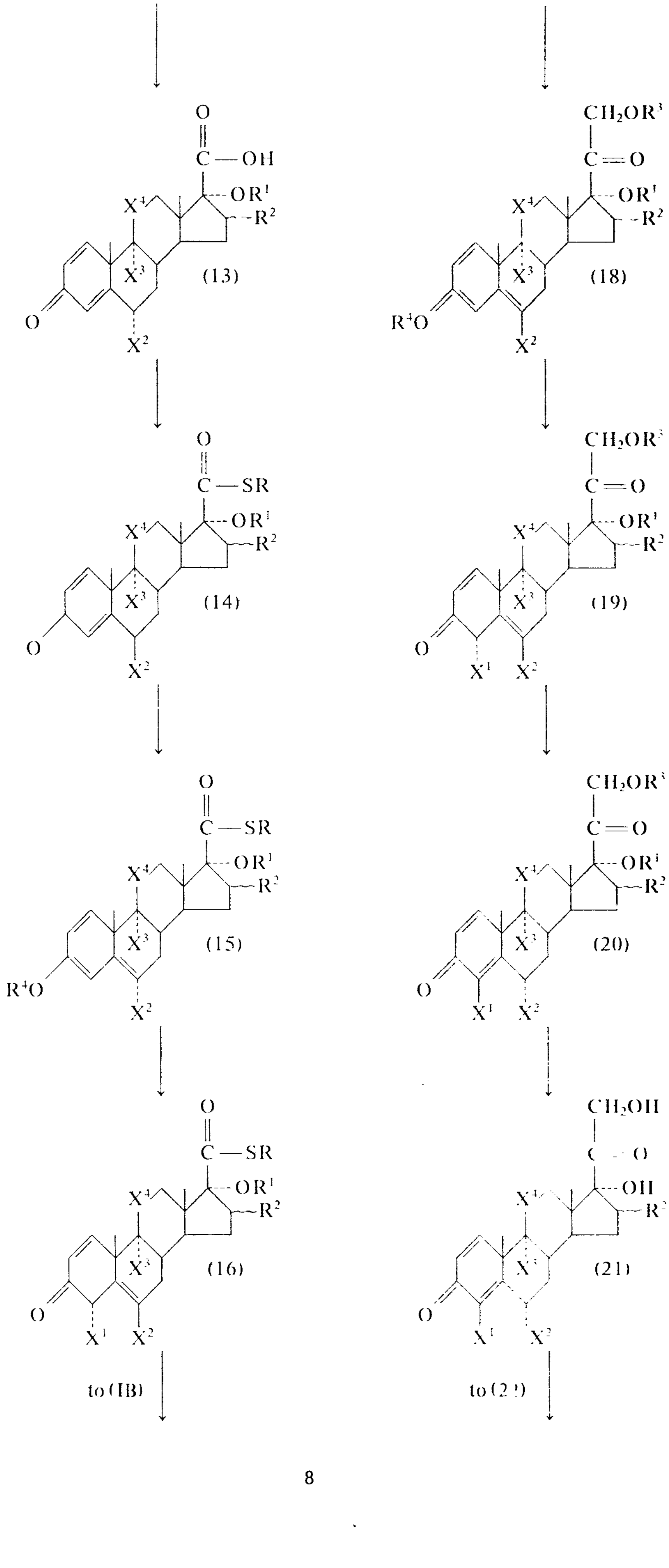

(13)

(18)

(14)

(19)

(15)

(20)

(16)

(21)

to (IB)

to (2?)

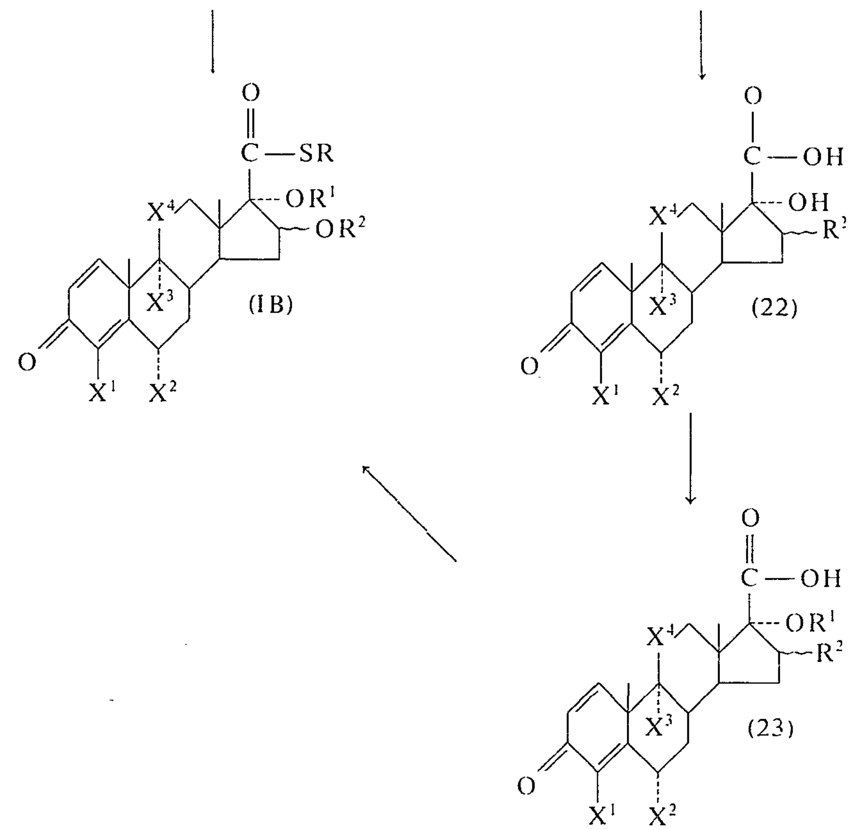

(IB) (22) (23)

Turning now to Reaction Sequence B, a process is shown for preparing the compounds of this invention wherein $R^1$ is alkanoyl, $R^2$ is $\alpha$-methyl, $\beta$-methyl or hydrogen; $X^3$ is fluoro, chloro or bromo; and $X^1$, $X^2$, $X^4$ and R are defined hereinbefore in the broad aspect of this invention, and $R^3$ and $R^4$ are as hereinbefore defined. The process is in essence the same as that set forth in Reaction Sequence A except that an alkanoyl, $R^1$, is introduced into formula (12) and (11) to form compounds of formulas (13) and (17), respectively. This is done by reacting a compound of formulas (12) or (11) with a suitable anhydride such as propionic anhydride, acetic anhydride, butyric anhydride, valeric anhydride, and the like in a suitable solvent such as a lower alkanol (e.g. methanol, ethanol) or an excess of anhydride itself in the presence of an organic base such as an amine (e.g. pyridine, triethylamine). In the case where $X^4$ is

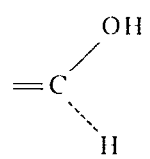

preferably sufficient anhydride (e.g. acetic anhydride) is reacted in the presence of ethylamine and dimethylaminopyridine to form an 11$\beta$,17$\alpha$,21-triacetoxy steroid of formula (17). The resulting compound is then haloganted at the 4-position as discussed for Reaction Sequence A, the 21 carbon is removed as discussed above and finally the alkyl, phenyl or benzyl 17$\beta$-thiocarboxylate (IB) is formed.

In the case where the compound represented by formula (20) is an 11$\beta$,17$\alpha$,21-triacetoxy compound, it is preferably first hydrolyzed using a weak base such as potassium carbonate in methanol at temperatures of about 10—50°C, e.g. ambient temperature, to form the 11$\beta$,17$\alpha$,21-trihydroxy compound of formula (21). This compound is reacted with potassium carbonate and oxygen to form a compound of formula (22) which is then alkanoated at 17$\alpha$ using propionic anhydride as discussed before to form a compound of formula (23). This then is reacted to form a compound of this invention represented by formula (IB) as discussed hereinbefore.

Alternatively, if the 21-carbon is removed first to form (12). This compound is then reacted with a suitable anhydride to form a compound of formula (13) which, in turn, is thioesterified to form a compound of formula (14) then halogenated to give the compound of the invention represented by formula (IB).

Again, once the 1,4-diene represented by formula (IB) is obtained, it is readily converted to the corresponding 4-ene by methods well known in the art such as hydrogenating with tris (triphenylphosphine) chlororhodium in ethanol.

Reaction Sequence C

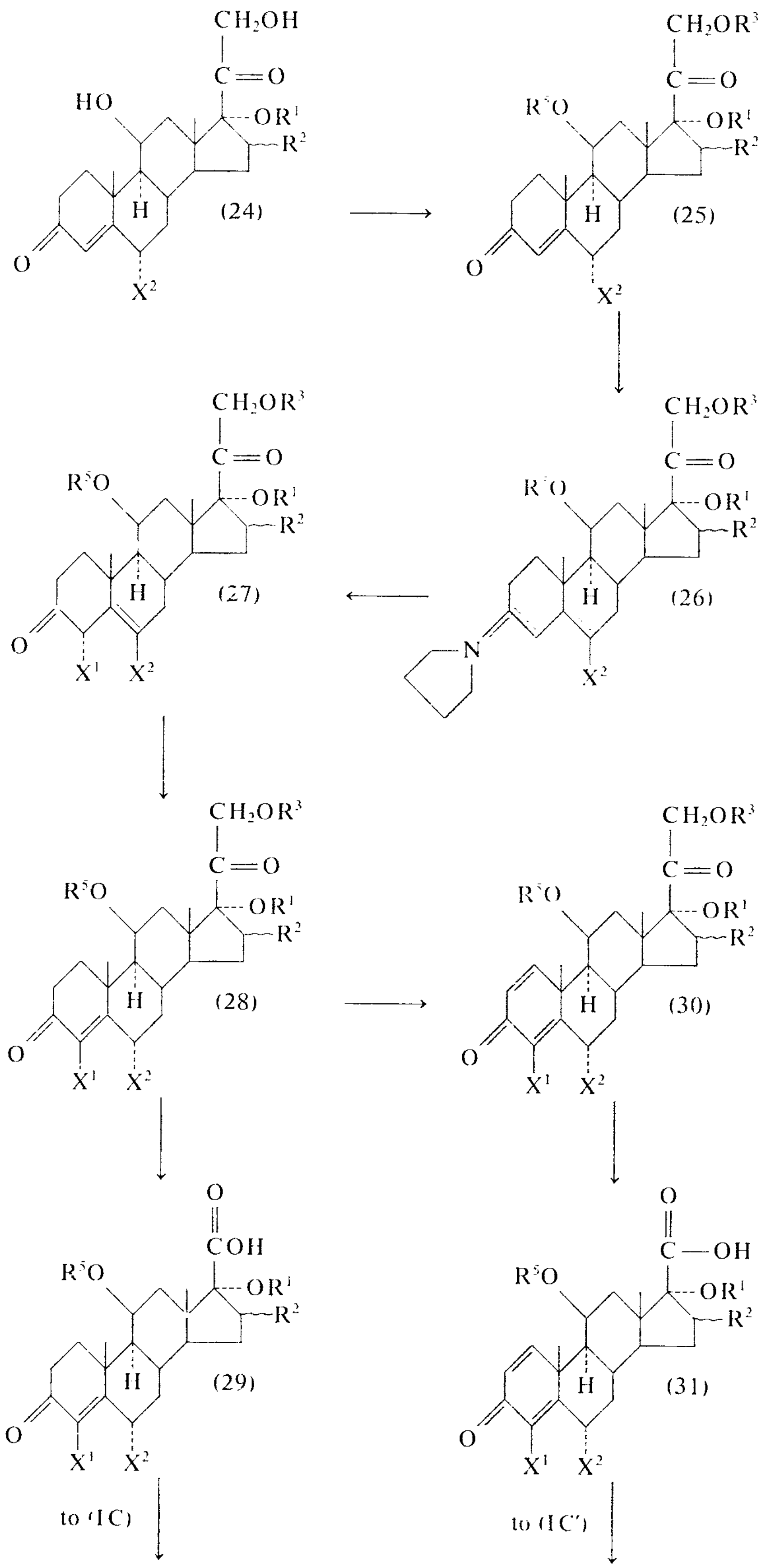


(24) (25) (26) (27) (28) (29) (30) (31)

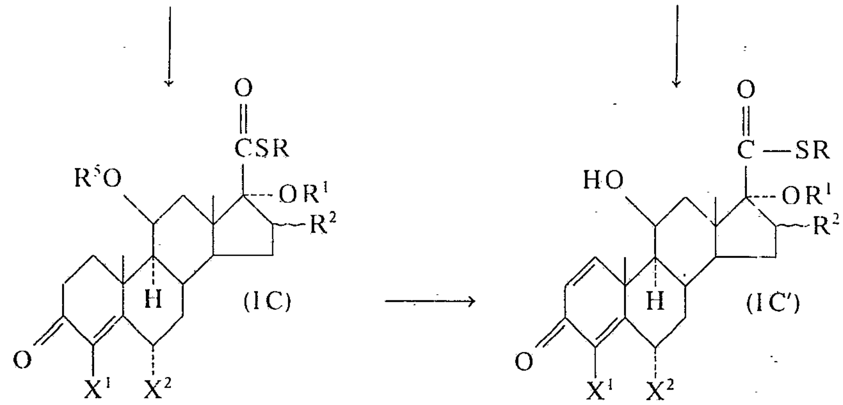

(IC) (IC')

If $X^3$ in the compound represented by formula (I) is hydrogen, then Reaction Sequence C is followed where the starting compound is represented by formula (24), wherein $OR^1$ and $R^2$ may be $16\alpha,17\alpha$-acetonide or $R^1$ is H or alkanoyl when $R^2$ is $\alpha$-methyl, $\beta$-methyl or hydrogen; $R^3$ and $R^5$ are alkanoyl; $X^1$ is fluoro, chloro or bromo; R is as hereinbefore defined; and $X^2$ is fluoro, chloro or hydrogen.

In the first step, if appropriate, the compound of formula (24) is alkanoated at $17\alpha$-, $11\beta$ and 21 as discussed hereinbefore. Thereafter the 4-halogenation proceeds through an intermediate enamine represented by formula (26) which is formed by reacting the compound of formula (25) with a suitable amine such as pyrrolidine, in an inert organic solvent such as benzene or methanol at temperatures of about 10° – 100° C, preferably about 50° – 55° C.

The other steps in the reacting sequence immediately above are in essence discussed hereinbefore. The conversion of a compound of formula (28) to one of formula (30) or (IC) to (IC') is performed by means known in the art such as by refluxing in dioxane and 2,3-dichloro-5,6-dicyano-1,4-benzoquinone for a sufficient period (generally less than 24 hours) to form the compound of formula (30) or (IC').

The starting materials for Reaction Sequences (A) – (C), represented by formulas (1), (11) or (24) are readily prepared by starting with compounds known in the art and proceeding according to known methods.

Suitable 21-hydroxy-3,20-dioxopregn-4-en or pregna-1,4-dienes include known compounds such as corticosterone, hydrocortisone, prednisolone, $\beta$methasone, dexamethasone, paramethasone, triamcinolone acetonide, fluocinolone acetonide, »and the like«. By following procedures generally known in the art steroids of a relatively simple structure can be converted to other structures as desired.

For example, the 6-fluoro or 6-chloro starting steroids can be prepared from known steroids such as 17-alpha-hydroxy-progesterone or hydrocortisone by treating a 3-methoxy-pregna-3,5-diene (prepared by reacting a 3-keto-pregn-4-ene with triethyl orthoformate in methanol) with perchloryl fluoride in dimethylformamide or dichlorhydantoin in acetone.

Other 6-fluoro starting steroids employed in the present process to prepared the novel $17\beta$-thiocarboxylic acid derivatives of this invention are described in the literature and in United States and foreign patents. For example, see U.S. Patents 2,983,737, 2,983,839, 3,053,838, 3,057,858, 3,124,251, 3,126,375, 3,201,391 and 3,248,389.

The $9\alpha$-fluoro, chloro or bromo group is introduced by treating a $9\alpha,11\beta$-oxido steroid with hydrogen fluoride, hydrogen chloride or hydrogen bromide respectively in an inert, nonaqueous, preferably anhydrous, solvent or mixture of such solvents. For example, see U.S. 3,211,758 to Tarkoey wherein a hydrogen fluoride/urea complex is employed. The $9\beta,11\beta$-oxido steroid is prepared from the corresponding pregna-1,4,9(11)-triene.

The pregna-1,4,9(11)-triene-3,20-diones or the corresponding 4,9(11)-dienes are known or can readily be prepared by treating a known 11-hydroxy steroid, such as prednisolone, $6\alpha$-fluoro-prednisolone or »the like«, in dimethylformamide and pyridine and reacting with methanesulfonyl chloride with about 5% sulfur trioxide at room temperature for 20 – 24 hours, then extracting with methylene chloride and recovering according to the process set forth in Example 3A of U.S. 3,009,933 to Robinson.

The pregna-1,4,9(11)-trienes or 4,9(11)-dienes are also converted to starting materials for Reaction Sequences (A), (B) or (C) by means known in the art. For example they are treated with chlorine according to the process of U.S. 3,009,933 to give the corresponding $9\alpha,11\beta$-dichloropregna-1,4-diene (i.e. $X^3$ is chloro and $X^4$ is

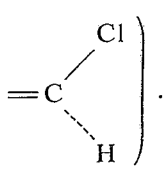

The 9α-bromo-11β-hydroxy compound or the 9α-chloro-11β-hydroxy compound is prepared by reacting the appropriate pregna-1,4,9(11) triene in dioxane with dibromohydantoin or dichlorohydantoin, respectively. This may be isolated and purified, or in turn, can be reacted with sodium hydroxide to give the corresponding 9β,11β-epoxide which is then treated with HCl, HBr or HF as discussed above.

An 11β-hydroxy (9-unsubstituted) steroid is readily prepared form a 3-keto-6α-substituted-pregna-1,4-diene or pregn-4-ene by methods well known in the art such as employing Cunninghamella blakesleeana, Cunninghamella bainieri, Curvularia lunata, or other suitable microorganisms in a suitable medium which selectively affords the desired 11β-hydroxy steroid.

The 16-methyl group is introduced by treating the corresponding 20-keto-16-pregnene with methyl magnesium bromide in the presence of cuprous chloride in an ether such as tetrahydrofuran. The 20-keto-16-pregnene steroid is prepared by preparing the 3,20-bis-semicarbazone of a 3,20-diketo-17α-hydroxy steroid, treating it with glacial acetic acid and acetic anhydride and then allowing the resulting product to react with aqueous pyruvic acid.

The 17α-hydroxy group is introduced in conjunction with the 16β-methyl group by first treating the corresponding 16-methyl-pregn-16-ene (which is prepared by treating the corresponding pregn-16-ene with diazomethane and then heating the resulting product to 180°C) with hydrogen peroxide, in an aqueous basic media, then permitting the resulting 16,17-oxido-16-methyl steroid to react with hydrogen bromide in glacial acetic acid. The resulting 16,17-bromohydrin is hydrogenated with the use of a palladium catalyst to afford the corresponding 16β-methyl-17-alpha-hydroxy derivative.

The 17α-hydroxy group is introduced in conjunction with the 16α-methyl by methods known in the art, such as the method described by Edwards et al in the Journal of the American Chemical Society 82, 2318—22, 1960. In this process an appropriate 21-substituted 16α-methylpregna-1,4-diene-3,2-dione is converted to 20-enol acetate by refluxing with acetic anhydride and freshly distilled acetyl chloride. The 20-enol acetate is recovered and reacted with monoperphthalic acid in ether and benzene to form the 17,20-epoxide which in turn is trated with methanol and aqueous potassium hydroxide to give the 16α-methyl-17α-hydroxy steroid which is isolated by means known in the art.

The compounds of this invention are useful for the relief of inflamed conditions in mammals, and more specifically are useful for relieving inflammatory manifestations of corticosteroid responsive dermatoses. Initial approximation of anti-inflammatory activity is done by following the procedure of McKenzie, S.W. and Stoughton, R.B., »Method for Comparing Percutaneous Absorption of Steroids« Arch Dermat, 86, 608 (1962) or modifications thereof.

Generally, the inflammatory manifestation in mammals, particularly humans, is combatted by treating the affliceted mammal with a therapeutically effective amount of the novel steroids of this invention, that is an amount which results in improvement of the inflamed condition. Preferably the steroids are first formulated to prepare a suitable pharmaceutical formulation, as discussed hereinafter, which is then placed in contact with the afflicted area. An effective amount will depend upon the particular condition and the mammal receiving the treatment but will vary between 0,001% to 10% by weight of the pharmaceutical omposition and preferably will be between 0,01 and 1% by weight of the formulation. Using these levels in the formulation, a therapeutically effective, non-side effect producing amount, i. e. enough to effect an anti-inflammatory response, but not enough to adversely effect the recipient, is applied to the inflamed area.

The compounds of this invention not only have antiinflammatory activity but also exhibit a low level of systemic activity, as measured by recognized laboratory assays. This allows for the application of an effective amount of the anti-inflammatory compounds with little adverse effect on the rest of the mammal's system.

The novel steroids of this invention may be formulated with suitable pharmaceutical excipients known in the art to form particularly effective anti-inflammatory compositions. Generally an effective amount of the steroid is about 0,001%w to about 10%w of the total formulated composition. The rest of the formulated composition will be about 90%w to about 99.999%w of at least one suitable excipient which may include a pharmaceutically acceptable solvent and ohter pharmaceutically acceptable additives to form an effective pharmaceutical formulation.

A pharmaceutically acceptable solvent is one which is substantially non-toxic and non-irritating under the conditions used and may be readily formulated into any of the classical drug formulations such as powders, creams, ointments, lotions, gels, foams, suppositories, aerosols, solutions or the like. Particularly suitable solvents include water, glycerine, propylene carbonate, and a glycol such as 1,2-propylene diol (i. e. propylene glycol), 1,3-propylene diol, polyethylene glycol having a molecular weight of from 100 to 10,000, dipropylene glycol, etc.; and mixtures of the aforementioned with each other.

A topical cream may be prepared as a semi-solid emulsion of oil in water or water in oil. A cream base formulation by definition is an emulsion which is a two-phase system with one liquid (for example fats or oils) being dispersed as small globules in another substance (e. q., a glycol-water solvent phase) which may be employed as the primary solvent for the novel steroids therein, the cream formulation may contain fatty alcohols, surfactants, mineral oil or petrolatum and other typical

pharmaceutical adjuvants such as anti-oxidants, antiseptics, or compatible adjuvants. A typical cream base formulation is given in the following table:

| | |
|---|---|
| Water/glycol mixture (15% or more glycol) | 50—99 parts by weight |
| Fatty alcohol | 1—20 |
| Non-ionic Surfactant | 0—10 |
| Mineral oil | 0—10 |
| Typical pharmaceutical adjuvants | 0—5 |
| Active Ingredients | 0.001—10 |

The fatty alcohol, non-ionic surfactant, and other adjuvants are discussed in U. S. 3,934,013 to Poulsen which is incorporated herein by reference.

The novel steroids of this invention may also be formulated as ointments. A »classical« ointment is a semisolid anhydrous composition which may contain mineral oil, white petrolatum, a suitable solvent such as a glycol and may include propylene carbonate and other pharmaceutically suitable additives such as surfactants, for example Span and Tween, or wool fat (lanolin), along with stabilizers such as antioxidants and other adjuvants as mentioned before. Following is an example of a typical »classical« ointment base:

| | |
|---|---|
| White petrolatum | 40—94 parts by weight |
| Mineral Oil | 5—20 |
| Glycol solvent | 1—15 |
| Surfactant | 0—10 |
| Stabilizer | 0—10 |
| Active Ingredients | 0,001—10.0 |

Other suitable ointment base formulations which employ propylene carbonate are described in U. S. patent 4,017,615 issued April 12, 1977 by Shastri et al entitled »Propylene Carbonate Ointment Vehicle« and U. S. 3,924,004 issued December 2, 1975 by Chang et al entitled »Fatty Alcohol-Propylene Carbonate-Glycol Solvent Cream Vehicle«. As much of those applications as is pertinent is incorporated herein by reference. Following is a typical ointment base formulation containing propylene carbonate:

| | |
|---|---|
| Active Ingredients | 0,001—10.0 parts by weight |
| Propylene Carbonate | 1—10 |
| Solvent | 1—10 |
| Surfactant | 1—10 |
| White Petrolatum | 70—97 |

Suitable solvents, surfactants, stabilizers, etc. are discussed in U. S. 3,934,013 and such discussion is incorporated herein by reference.

A suitable »non-classical« anhydrous, water washable »ointment type« base is described in U. S. Patent No. 3,952,930 to Katz and Neiman, and that patent is incorporated herein by reference. A representative composition of this invention utilizing such a base is as follows:

| | |
|---|---|
| Glycol solvent | 40—35 parts by weight |
| Fatty alcohol | 15—45 |
| Compatible plasticizer | 0—15 |
| Compatible coupling Agent | 0—15 |
| Penetrant | 0—20 |
| Active Ingredients | 0,001—10.0 |

Preparation I

This preparation sets forth a process for preparing 17$\beta$-carboxylic acid precursors of compounds represented by formula (I) wherein

$X^1$ is fluoro;
$X^2$ is fluoro, chloro or hydrogen;
$X^3$ is fluoro, chloro or bromo;

$X^4$ is

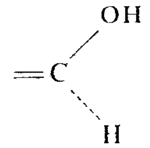

or is

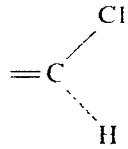

when $X^3$ is Cl;

$R^1$ is alkanoyl of 2—6 carbon atoms; and

$R^2$ is hydrogen or methyl.

A. Ten grams (10 g.) of flumethasone is treated at room temperature with 50 ml of $Et_3N$ and 50 ml of acetic anhydride plus 2 g of dimethylaminopyridine. The mixture is heated on the steam bath for 5 hours. Examination of the reaction mixture by thin layer chromatography (TLC) analysis using 10% ethyl acetate/90% dichloromethane (DCM) shows the reaction to be complete. The mixture is cooled in an ice water bath, and slowly diluted with water up to a final volume of 2 liters. The semicrystalline precipitate so obtained is collected by filtration, washed with water, and dissolved in 200 ml of DCM, dried over anhydrous sodium sulfate, filtered and concentrated to dryness. The residue is dissolved in DCM and the solution is passed through a column of 100 g of silica gel eluting with 100% DCM, then 4% ethyl acetate in DCM. The homogeneous fractions are combined and concentrated to dryness. The residue is crystallized from $CH_2Cl_2$/MeOH and filtered. The filtrate is dried on the steam bath to give 11β,17α,21-triacetoxy-6α,9α-difluoro-16α-methylpregna-1,4-diene-3,20-dione.

Ten g of the triacetate prepared in this manner is treated with 150 ml of trimethylorthoformate and 50 ml of anhydrous methanol, using 5 ml of fuming sulfuric acid as catalyst. The reaction mixture is heated at 40—50°C for a period of 30 minutes, then 25 ml of triethylamine (TEA) is added, and the mixture concentrated under reduced pressure to dryness. The residue is dissolved into 200 ml of DCM, washed thrice with 50 ml of water, dried over anhydrous sodium sulfate, filtered, and the solvent removed under reduced pressure. The residue is dissolved in 25 mls of pyridine and treated at room temperature with 5 ml of acitic anhydride for a period of one hour. The reaction mixture is diluted slowly with 500 mls of water, stirred at room temperature for 4 hours. The precipitate so obtained is collected by filtration, washed with water, dissolved in 200 ml of DCM, dried over anhydrous sodium sulfate, filtered and concentrated to dryness. The residue is chromatographed over 100 g of silica gel in a DCM/hexane system to give 7.5 g of 11β,17α,21-triacetoxy-6,9α-difluoro-16α-methyl-3-methoxypregna-1,3,5-triene-20-one, after concentration of the homogenous fractions.

A mixture of 10 g of the trienol ether prepared in this manner in 300 ml of 90% acetone/10% water, is treated at room temperature with a slow stream of perchloryl fluoride for a period of 45 minutes. The reaction mixture is diluted with 300 ml of water, and the acetone is eliminated under reduced pressure. The precipitate so obtained is extracted with DCM, washed thrice with 50 ml of water, dried over anhydrous sodium sulfate, and concentrated to dryness under reduced pressure. The residue is percolated through a column of 100 g silica gel eluting with a DCM/hexane system; increasing the polarity of the eluant to 100% DCM gradually. The homogeneous fractions containing 11β,17α,21-triacetoxy-4α,6,9α-trifluoro-16α-methyl-1,5-diene-3,20-dione are combined, concentrated to dryness to give 5.3 g of 11β,17α,21-triacetoxy-4α,6,9α-trifluoro-16α-methyl-1,5-diene-3,20-dione.

Ten g of 11β,17α,21-triacetoxy-4α,6,9α-trifluoro-16α-methylpregna-1,5-diene-3,20-dione prepared in this manner in 300 ml of methanol is treated at room temperature with 2 g of anhydrous potassium carbonate under nitrogen for 1 hour. The mixture is acidified by addition of 10 ml of glacial acetic acid; the mixture is diluted with 300 ml of water; and the methanol eliminated under reduced pressure, to leave a crystalline precipitate of 4α,6,9α-trifluoro-11β,17α,21-trihydroxy-16α-methylpregna-1,4-diene-3,20-dione.

Ten (10) g of 4,6α,9α-trifluoro-11β,17α,21-trihydroxy-16α-methylpregna-1,4-diene-3,20-dione in 300 ml of anhydrous methanol is treated with 30 g of anhydrous potassium carbonate at room temperature under stirring for a period of 24 hours while air is continuously bubbled through the reaction mixture. Methanol is added at intervals to maintain the original volume. The reaction mixture is diluted with 300 mls of water, and acidified with concentrated hydrochloric acid until a pH of 2 is obtained. The reaction mixture is concentrated under reduced pressure, until most of the methanol is eliminated. The mixture is cooled to room temperature, and the resulting crystalline precipitate is collected by filtration and air dried to yield 4,6α,9α-trifluoro-11β,17α-dihydroxy-16α-methyl-3-oxoandrosta-1,4-diene-17β-carboxylic acid.

Ten (10) g of 4,6α,9α-trifluoro-11β,17α-dihydroxy-16α-methyl-3-oxoandrosta-1,4-diene 17β-carboxylic acid is treated at room temperature with 50 ml of propionic anhydride and 50 mls of anhydrous pyridine. The mixture is stirred for one hour, then slowly diluted with water up to 2 liters while the mixture is cooled in an ice-water bath. The crystalline precipitate so obtained is collected by filtration, washed with water and dried, to give 4,6α,9α-trifluoro-11β-hydroxy-16α-methyl-3-oxo-17α-propionyloxyandrosta-1,4-diene 17β-carboxylic acid.

Similarly, by substituting an appropriate starting material for flumethasone the following compounds are prepared:

9α,11β-dichloro-4,6α-difluoro-16α-methyl-3-oxo-17α-propionyloxyandrosta-1,4-diene 17β-carboxylic acid;
9α-bromo-4,6α-difluoro-11β-hydroxy-16α-methyl-3-oxo-17α-propionyloxyandrosta-1,4-diene 17β-carboxylic acid;
9α-chloro-4,6α-difluoro-11β-hydroxy-16α-methyl-3-oxo-17α-propionyloxyandrosta-1,4-diene 17β-carboxylic acid;
6α,9α-dichloro-4-fluoro-11β-hydroxy-16α-methyl-3-oxo-17α-propionyloxyandrosta-1,4-diene 17β-carboxylic acid;
9α-bromo-6α-chloro-4-fluoro-11β-hydroxy-16α-methyl-3-oxo-17α-propionyloxy-androsta-1,4-diene 17β-carboxylic acid;
4,9α-difluoro-11β-hydroxy-16α-methyl-2-oxo-17α-propionyloxyandrosta-1,4-diene 17β-carboxylic acid;
9α-chloro-4-fluoro-11β-hydroxy-16α-methyl-3-oxo-17α-propionyloxyandrosta-1,4-diene 17β-carboxylic acid; and
9α-bromo-4-fluoro-11β-hydroxy-16α-methyl-3-oxo-17α-propionyloxyandrosta-1,4-diene 17β-carboxylic acid.

C. By substituting acetic anhydride, n-butyric anhydride, valeric anhydride, or caproic anhydride for propionic anhydride, the corresponding 17α-acetates, -n-butyrates, valerates or caproates are prepared, e. g.

17α-acetoxy-4,6α,9α-trifluoro-11β-hydroxy-16α-methyl-3-oxoandrosta-1,4-diene 17β-carboxylic acid;
17α-butyroxy-4,6α,9α-trifluoro-11β-hydroxy-16α-methyl-3-oxoandrosta-1,4-diene 17β-carboxylic acid;
17α-caproyloxy-4,6α,9α-trifluoro-11β-nydroxy-16α-methyl-3-oxoandrosta-1,4-diene 17β-carboxylic acid;
4,6α,9α-trifluoro-11β-hydroxy-16α-methyl-3-oxo-17α-valeryloxyandrosta-1,4-diene 17β-carboxylic acids;
and the linke.

D. The above compounds wherein $R^2$ is β-methyl or H are prepared from the starting materials having a 16β-methyl substituent or being unsubstituted at 16, e. g.

17α-acetoxy-4,6α,9α-trifluoro-11β-hydroxy-16β-methyl-3-oxoandrosta-1,4-diene 17β-carboxylic acid;
17α-butyroxy-4,6α,9α-trifluoro-11β-hydroxy-16β-methyl-3-oxoandrosta-1,4-diene 17β-carboxylic acid;
17α-caproyloxy-4,6α,9α-trifluoro-11β-hydroxy-16β-methyl-3-oxoandrosta-1,4-diene 17β-carboxylic acid;
4,6α,9α-trifluoro-11β-hydroxy-16β-methyl-3-oxo-16α-propionyloxyandrosta-1,4-diene 17β-carboxylic acid;
and the like.

Preparation II

This preparation sets forth a process for preparing 17β-carboxylic acid precursors of compounds represented by Formula (I) wherein

$X^1$ is fluoro;
$X^2$ is fluoro or hydrogen;
$X^3$ is fluoro, chloro or bromo;
$X^4$ is

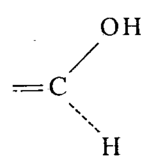 or is 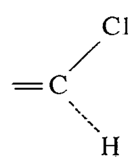

when $X^3$ is Cl; and
$OR^1$ and $R^2$ together are 16α,17α-acetonide.

A. By substituting fluocinolone acetonide for flumethasone in Preparation I, Part 1, and following in principle that process, 4,6α,9α-trifluoro-11β-hydroxy-16α,17α-isopropylidenedioxy-3-oxo-androsta-1,4-diene 17β-carboxylic acid is prepared.

B. Similarly by substituting an appropriate starting material for fluocinolone acetonide in Part A of this preparation, other compounds are prepared such as

9α,11β-dichloro-4,6α-difluoro-16α,-17α-isopropylidenedioxy-3-oxoandrosta-1,4-diene 17β-carboxylic acid;
4,9α-difluoro-11β-hydroxy-16α,17α-isopropylidene-dioxy-3-oxoandrosta-1,4-diene 17β-carboxylic acid;
9α-chloro-4-fluoro-11β-hydroxy-16α,17α-isopropylidenedioxy-3-oxoandrosta-1,4-diene 17β-carboxylic acid;
and the like.

## Preparation III

This preparation sets forth a process for preparing 17β-carboxylic acid precursors of compounds represented by Formula (I) wherein $X^1$ is chloro or bromo and $X^2$, $X^3$, $X^4$, $R^1$ and $R^2$ are the same as in Preparation I.

A. By following in principle the procedure of Preparation I, but substituting dichlorohydantoin or dibromohydantoin in aqueous acetone for perchloryl fluoride, the following compounds are prepared:

4-chloro-6α,9α-difluoro-11β-hydroxy-16α-methyl-3-oxo-17α-propionyloxyandrosta-1,4-diene 17β-carboxylic acid;
4,9α,11β-trichloro-6α-fluoro-16α-methyl-3-oxo-17α-n-propionyloxyandrosta-1,4-diene 17β-carboxylic acid;
4-chloro-9α-fluoro-11β-hydroxy-16α-methyl-3-oxo-17α-n-propionyloxyandrosta-1,4-diene 17β-carboxylic acid;
4,9α-dichloro-11β-hydroxy-16α-methyl-3-oxo-17α-propionyloxyandrosta-1,4-diene 17β-carboxylic acid;
the analogous 4-bromo steroids;
and the like.

B. By following in principle the procedure of Preparation I, Part B, other 17α-alkanoyloxy derivatives of the compounds of Part A ot this Example such as the 17α-acetates, 17α-n-butyrates, 17α-valerates or 17α-caproates.

C. The compounds of Parts A and B of this preparation wherein $R^2$ is β-methyl or H are prepared from starting materials having a 16β-methyl or being unsubstituted at the 16 position.

## Preparation IV

This preparation sets forth a process for preparing 17β-carbonylic acid precursors of compounds represented by Formula (I) wherein $X^1$ is chloro or bromo and $X^2$, $X^3$, $X^4$, $R^1$ and $R^2$ are the same as in Preparation II.

By following in prinziple the procedure of Preparation II, Parts A and B but substituting dichlorohydantoin or dibromohydantoin in aqueous acetone for perchloryl fluoride the following compounds are prepared:

4,9α-dichloro-6α-fluoro-11β-hydroxy-16α,17α-isopropylidenedioxy-3-oxoandrosta-1,4-diene 17β-carboxylic acid;
4-chloro-6α,9α-difluoro-11β-hydroxy-16α,17α-isopropylidenedioxy-3-oxoandrosta-1,4-diene 17β-carboxylic acid;
4,9α,11β-trichloro-6α-fluoro-16α,17α-isopropylidenedioxy-3-oxoandrosta-1,4-diene 17β-carboxylic acid;
4,6α,9α-trichloro-11β-hydroxy-16α,17α-isopropylidenedioxy-3-oxoandrosta-1,4-diene 17β-carboxylic acid;
the analogous 4-bromo steroids;
and the like.

Preparation V

This preparation sets forth a process for preparing 17β-carboxylic acid precursors of compounds represented by Formula (I) wherein

$X^1$ is fluoro, chloro or bromo;
$X^2$ is fluoro, chloro or hydrogen;
$X^3$ is hydrogen;
$X^4$ is

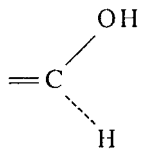

$R^1$ is alkanoyloxy of 2—6 carbons; and
$R^2$ is hydrogen, α-methyl or β-methyl; and
there is a single bond between C-1 and C-2.

A. Twenty g. of paramethasone (6α-fluoro-16α-methyl-prednisolone) are dehydrogenated according to methods discussed herein to give 6α-fluoro-16α-methyl-hydrocortisone.

Ten g. of a compound made according to the process of the preceeding paragraph are added to a mixture of and about 5 g of pyrrolidine for a period of 5 to 24 hours. When TLC analysis shows the reaction is complete, the solvents are eliminated under reduced pressure to leave the enamine 6-fluoro-11-beta,17α,21-trihydroxy-16α-methyl-3-pyrrolidinyl-pregna-3,5-diene-20-one. The enamine is then purified by dissolving in a suitable organic solvent, filtering and recrystallizing or by column chromatography using alumina.

One g of a purified »enamine« from the previous step is dissolved in about 20 ml pyridine and the resulting solution is cooled to 0° C. A current of perchloryl fluoride is slowly bubbled into the stirred mixture and the reaction is monitored by TLC analysis. When analysis indicates the reaction is complete, the mixture is concentrated under reduced pressure to give a crude residue of 4α,6-difluoro-11β,17α,21-trihydroxy-16α-methylpregna-5-ene-3,20-dione. The crude product is purified by recrystallization from a suitable solvent or by chromatography on silica gel.

One-half (0,5) g of the compound prepared according to the previous paragraph is dissolved in 20 ml of ethanol under a nitrogen atmosphere. A catalytic amount (approximately 20% of an equivalent based on the steroid) of anhydrous potassium carbonate is added, and the mixture stirred at room temperature for 1 to 5 hours while the reaction is monitored by TLC analysis. When the analysis indicates that the reaction is complete, glacial acetic acid is added to neutralize the potassium carbonate. Then the mixture is concentrated under reduced pressure to a small volume and diluted with water, to give a crystalline precipitate of 4,6α-difluoro-11β,17α,21-trihydroxy-16α-methylpregn-4-ene-3,20-dione.

250 Mg of the resulting product in turn is mixed with 10 ml methanol and 50 mg anhydrous potassium carbonate and stirred at ambient temperature and atmospheric pressure while a current of air is slowly bubbled through the reaction mixture for 22 hours. Methanol is added at periodic intervals to maintain a constant volume. The reaction mixture is diluted with water to give a total volume of 300 ml, then concentrated hydrochloric acid is added slowly while stirring until a pH of 2 is obtained. The resulting crystalline precipitate is collected by filtration and air dried to give 4,6α-difluoro-11β,17α-dihydroxy-16α-methyl-3-oxoandrost-4-ene-17β-carboxylic acid.

Similarly, by following in principle the procedure of this Preparation, above, but substituting dichlorohydantoin or N-bromosuccinimide for perchloryl fluoride, the following compounds are obtained:

4-chloro-6α-fluoro-11β,17α-dihydroxy-16-alpha-methyl-3-oxoandrost-4-ene 17β-carboxylic acid; and
4-bromo-6α-fluoro-11β,17α-dihydroxy-16α-methyl-3-oxoandrost-4-ene 17β-carboxylic acid.
4-fluoro-11β,17α-dihydroxy-16α-methyl-3-oxoandrost-4-diene 17β-carboxylic acid;
and the like.

B. By reacting each of the resulting products with acetic anhydride, propionic anhydride, n-butyric anhydride, valeric anhydride or caproic anhydride as discussed in Parts A and C of Preparation I, the corresponding 17α-acetate, -propionate, -n-butyrate, valerate and caproate are preprepared.

C. The compounds of Part A and B of this preparation wherein $R^2$ at the 16 position is 16β-methyl or hydrogen are prepared from the starting materials having a 16β-methyl substituent or being unsubstituted at 16.

## Preparation VI

This preparation sets forth a process for preparing 17β-carboxylic acid precursors of compounds of formula (I) wherein $OR^1$ and $R^2$ together are 16α,17α-isopropylidenedioxy and $X^1$, $X^2$, $X^3$ and $X^4$ are the same as in Preparation V.

A. By following in principle the process of Preparation V, Part A but substituting 6α-fluoro-11β-hydroxy-16α,17α-isopropylidenedioxypregna-1,4-diene-3,20-dione for paramethasone, 4,6α-difluoro-11β-hydroxy-16α,17α-isopropylidenedioxy-3-oxo-androst-4-ene 17β-carboxylic acid is prepared.

By substituting dichlorohydantoin or dibromohydantoin for perchloryl fluoride in this procedure the following compounds are prepared:

4-chloro-6α-fluoro-11β-hydroxy-16α,17α-isopropylidenedioxy-3-oxoandrost-4-ene 17β-carboxylic acid;
4-bromo-6α-fluoro-11β-hydroxy-16α,17-alpha-isopropylidenedioxy-3-oxoandrost-4-ene 17β-carboxylic acid;
4-fluoro-11β-hydroxy-16α,17α-isopropylidenedioxy-3-oxoandrost-4-ene 17β-carboxylic acid;
and the like.

Specific embodiments of the process for preparing compunds of this invention are found in the following Examples which are given by way of illustration only and are not be be interpreted as limiting the scope of the claims appended hereto.

## Example 1

This example sets forth a process for preparing compounds of this invention represented by formula (I) wherein; R is alkyl, benzyl or phenyl; and $X^2$, $X^3$, $X^4$, $R^1$ and $R^2$ are defined in Preparation I.

A. Six hundred (600) mg of 4,6α,9α-trifluoro-11β-hydroxy-16α-methyl-3-oxo-17α-propionyloxyandrosta-1,4-diene 17-beta-carboxylic acid (prepared as set forth in Preparation I), 8 milliliters (ml) of tetrahydrofuran (THF) and 0.2 ml triethylamine (TEA) are placed in a suitable reaction vessel. The mixture is stirred at room temperature under nitrogen and 240 mg of diethyl chlorophosphate (DCP: $C_2H_5O)_2P(O)Cl$) in 8 ml of THF is added thereto. The reaction mixture is stirred under nitrogen at room temperature for six hours whereupon 0.04 ml of TEA is added followed by 0.05 g DCP in 3 ml of THF. The resulting mixture is stirred for an additional 17.5 hours. The resulting precipitate is filtered off and washed with 10 ml THF. A solution of 2.05 ml of a solution of 20 ml DMF, 0.758 g 57% sodium hydride and 0.86 methyl sulfide (MeS) is added to the filtrate and the resulting mixture is stirred for about 5.5 hours at which time another 1 ml of the DMF/NaH/MeS solution is added.

This mixture is poured into 200 ml ethyl acetate, washed twice with 200 ml water, washed with brine, dried over sodium sulfate, filtrered, and the solvents removed by rotary evaporator to give a crude material which is then purified by recrystallizing with acetone/-hexane to give methyl 4,6α,9α-trifluoro-11β-hydroxy-16α-methyl-3-oxo-17α-propionyloxyandrosta-1,4-diene 17β-thiocarboxylate, m.p. 287.5–290°C.

B. Similarly, by following the above procedure in this example but substituting other starting materials from Preparation I for 4,6α,9α-trifluoro-11β-hydroxy-16α-methyl-3-oxo-17α-propionyloxyandrosta-1,4-diene 17β-carboxylic acid and/or other alkyl, benzyl or phenyl sulfides for methyl sulfide, other compounds of this invention are prepared, such as

methyl 9α,11β-dichloro-4,6α-difluoro-16α-methyl-3-oxo-17α-propionyloxyandrosta-1,4-diene 17β-thiocarboxylate;
n-hexyl-17α-acetoxy-chloro-6α-fluoro-11β-hydroxy-16α-methyl-3-oxo-androsta-1,4-diene 17β-thiocarboxylate;
methyl 17α-caproyl-4,6α,9α-trifluoro-11β-hydroxy-16α-methyl-3-oxoandrosta-1,4-diene 17β-thiocarboxylate;
ethyl 9α,11β-dichloro-4,6α-difluoro-16α-methyl-3-oxo-17α-propionyloxyandrosta-1,4-diene 17β-thiocarboxylate;
ethyl 4,6α,9α-trifluoro-11β-hydroxy-16α-methyl-3-oxo-17α-n-propionyloxyandrosta-1,4-diene 17β-thiocarboxylate;
benzyl 4,6α,9β-trifluoro-11β-hydroxy-16α-methyl-3-oxo-17α-propionyloxyandrosta-1,4-diene 17β-thiocarboxylate;
phenyl-4,6α,9α-trifluoro-11β-hydroxy-16α-methyl-3-oxo-17α-propionyloxyandrosta-1,4-diene 17β-thiocarboxylate;
4-chlorobenzyl 17α-acetoxy-4,6α,9α-trifluoro-11β-hydroxy-16α-methyl-3-oxo-androsta-1,4-diene 17β-thiocarboxylate; »and the like«.

Example 2

This example sets forth a process for preparing compounds of this invention represented by formula (I) wherein; R is alkyl, benzyl or phenyl; and $X^1$, $X^2$, $X^3$, $X^4$, $R^1$ and $R^2$ are as defined in Preparation II.

A. By following in principle the process of Example 1, Part A, but substituting 4,6α,9α-trifluoro-11β-hydroxy-16α,17α-isopropylidenedioxy-3-oxoandrosta-1,4-diene 17β-carboxylic acid, prepared in the manner set forth in Part A of Preparation 1, for 4,6α,9α-trifluoro-11β-hydroxy-16α-methyl-3-oxo-17α-propionyloxyandrosta-1,4-diene 17β-carboxylic acid, the compound methyl 4,6α,9α-trifluoro-11β-hydroxy-16α,17α-isopropylidenedioxy-3-oxoandrosta-1,4-diene 17β-thiocarboxylate, m. p. 284—287° C.

B. By following in principle the process of Part A of this example but substituting other alkyl, benzyl or phenyl sulfides for methyl sulfide and other 16α,17α-acetonides prepared according to the process of Preparation II for 4,6α,9α-trifluoro-11β-hydroxy-16α,17α-isopropylidenedioxy-3-oxo-androsta-1,4-diene 17β-carboxylic acid, other compounds of this invention are prepared such as

ethyl 4,6α,9α-trifluoro-11β-hydroxy-16α,17α-isopropylidenedioxy-3-oxoandrosta-1,4-diene 17β-thiocarboxylate;
isopropyl 4,6α,9α-trifluoro-11β-hydroxy-16α,17α-isopropylidenedioxy-3-oxoandrosta-1,4-diene 17β-thiocarboxylate;
benzyl 4,6α,9α-trifluoro-11β-hydroxy-16α,17α-isopropylidenedioxy-3-oxoandrosta-1,4-diene 17β-thiocarboxylate;
phenyl 4,6α,9α-trifluoro-11β-hydroxy-16α,17α-isopropylidenedioxy-3-oxoandrosta-1,4-diene 17β-thiocarboxylate;
methyl 9α,11β-dichloro-4,6α-difluoro-16α,17α-isopropylidenedioxy-3-oxoandrosta-1,4-diene thiocarboxylate;
n-hexyl-4,6α,9α-trifluoro-11β-hydroxy-16α,17α-isopropylidenedioxy-3-oxoandrosta-1,4-diene 17β-thiocarboxylate; m. p. 270—272° C;
pentyl 9α,11β-dichloro-4,6α-difluoro-16α,17α-isopropylidenedioxy-3-oxoandrosta-1,4-diene thiocarboxylate;
benzyl 9α,11β-dichloro-4,6α-difluoro-16α,17α-isopropylidenedioxy-3-oxoandrosta-1,4-diene thiocarboxylate.

Example 3

This example sets forth a process for preparing compounds of this invention represented by Formula (I) wherein; R is alkyl, phenyl or benzyl; and $X^1$, $X^2$, $X^3$, $X^4$, $R^1$ and $R^2$ are as defined in Preparation III.

By following in principle the process of Example 1, but substituting an appropriate starting material from Preaparation III for 4,6α,9α-trifluoro-11β-hydroxy-16α-methyl-3-oxo-17α-propionyloxyandrosta-1,4-diene 17β-carboxylic acid and optionally substituting other alkyl, phenyl or benzyl sulfides for methyl sulfide, other alkyl, phenyl or benzyl 17β-thiocarboxylates are prepared such as

methyl 4,9α,11β-trichloro-6α-fluoro-16α-methyl-3-oxo-17α-propionyloxyandrosta-1,4-diene 17β-thiocarboxylate;
methyl 4-chloro-6α,9α-difluoro-11β-hydroxy-16α-methyl-3-oxo-17α-propionyloxy-androsta-1,4-diene 17β-thiocarboxylate;
ethyl 17α-acetoxy-4-chloro-6α,9α-difluoro-11β-hydroxy-16α-methyl-3-oxoandrosta-1,4-diene 17β-thiocarboxylate;
benzyl 4,9α,11β-trichloro-6α-fluoro-16α-methyl-3-oxo-17α-propionyloxyandrosta-1,4-diene 17β-thiocarboxylate;
phenyl 17α-butyryloxy-4,9α,11β-trichloro-6α-fluoro-16α-methyl-3-oxoandrosta-1,4-diene 17 beta-thiocarboxylate;
n-propyl-4-chloro-6α,9α-difluoro-11β-hydroxy-16α-methyl-3-oxo-17α-propionyloxyandrosta-1,4-diene 17β-thiocarboxylate;
n-hexyl 4,9α,11β-trichloro-6α-fluoro-16α-methyl-3-oxo-17α-propionyloxyandrosta-1,4-diene 17β-thiocarboxylate;
n-pentyl 17α-acetoxy-4-chloro-6α,9α-difluoro-11β-hydroxy-16α-methyl-3-oxoandrosta-1,4-diene 17β-thiocarboxylate; and the like.

Example 4

This example sets forth a process for preparing compounds of this invention represented by Formula (I) wherein; R is alkyl, benzyl or phenyl; and $X^1$, $X^2$, $X^3$, $X^4$, $OR^1$ and $R^2$ are defined in Preparation IV.

By following in principle the process of Example 2 but substituting an appropriate 16α,17α-acetonide

for 4,6α,9α-trifluoro-11β-hydroxy-16α,17α-isopropylidenedioxy-3-oxoandrosta-1,4-diene 17β-carboxylic acid and optionally substituting other alkyl, phenyl or benzyl sulfides for methyl sufide, other compounds of this invention are prepared such as

methyl 4-chloro-6α,9α-difluoro-11β-hydroxy-16α,17α-isopropylidenedioxy-3-oxoandrosta-1,4-diene 17β-thiocarboxylate;
ethyl 4-chloro-6α,9α-difluoro-11β-hydroxy-16α,17α-isopropylidenedioxy-3-oxoandrosta-1,4-diene 17β-thiocarboxylate;
phenyl 4-chloro-6α,9α-difluoro-11β-hydroxy-16α,17α-isopropylidenedioxy-3-oxoandrosta-1,4-diene 17β-thiocarboxylate;
methyl 4,9α,11β-trichloro-6α-fluoro-16α,17α-isopropylidenedioxy-3-oxoandrosta-1,4-diene 17β-thiocarboxylate;
ethyl 4,9α,11β-trichloro-6α-fluoro-16α,17α-isopropylidenedioxy-3-oxoandrosta-1,4-diene 17β-thiocarboxylate;
isopropyl 4,9α,11β-trichloro-6α-fluoro-16α,17α-isopropylidenedioxy-3-oxoandrosta-1,4-diene 17β-thiocarboxylate;
benzyl 4,9α,11β-trichloro-6α-fluoro-16α,17α-isopropylidenedioxy-3-oxoandrosta-1,4-diene 17β-thiocarboxylate.

## Example 5

This example sets forth a process for preparing the compounds of this invention represented by Formula (I) wherein; $X^3$ is hydrogen; $X^4$ is

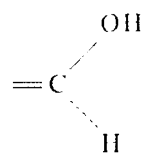

R is alkyl, benzyl or phenyl, and $X^1$, $X^2$, $R^1$ and $R^2$ are defined in Preparation V.

By following in principle the process of Example I, but substituting an appropriate starting material from Preparation V for 4,6α,9α-trifluoro-11β-hydroxy-17α-methyl-3-oxo-17α-propionyloxy-androst-1,4-diene 17β-carboxylic acid and optionally substituting other alkyl, phenyl or benzyl sulfides for methyl sulfide, other alkyl, phenyl or benzyl 17β-thiocarboxylates are prepared such as

methyl 4-chloro-6α-fluoro-11β-hydroxy-16α-methyl-3-oxo-17α-propionyloxyandrost-4-ene 17β-thiocarboxylate;
ethyl 4,6α-difluoro-11β-hydroxy-16α-methyl-3-oxo-17α-propionyloxyandrost-4-ene 17β-thiocarboxylate;
n-propyl 4-fluoro-11β-hydroxy-16α-methyl-3-oxo-17α-propionyloxyandrost-4-ene 17β-thiocarboxylate;
n-pentyl 4,6α-difluoro-11β-hydroxy-16α-methyl-3-oxo-17α-propionyloxyandrost-4-ene 17β-thiocarboxylate;
benzyl 4,6α-difluoro-11β-hydroxy-16α-methyl-3-oxo-17α-propionyloxyandrost-4-ene 17β-thiocarboxylate;
phenyl 4,6α-difluoro-11β-hydroxy-16α-methyl-3-oxo-17α-propionyloxyandrost-4-ene 17β-thiocarboxylate; and the like.

## Example 6

This example sets forth a process for preparing com pounds of this invention represented by Formula (I) wherein; R is alkyl, benzyl or phenyl; $X^3$ is hydrogen; $X^4$ is

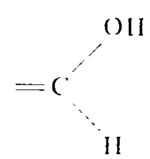

and $X^1$, $X^2$, $R^1$ and $R^2$ are defined in Preparation VI.

By following in principle the process of Example 2, but substituting an appropriate starting material from Preparation VI for 4,6α,9α-trifluoro-11β-hydroxy-16α,17α-isopropylidenedioxy-3-oxoandrosta-1,4-diene 17β-carboxylic acid and optionally substituting other alkyl, phenyl or benzyl sulfides for methyl sulfide, other alkyl, phenyl or benzyl 17β-thiocarboxylates are prepared such as

methyl 4,6α-difluoro-11β-hydroxy-16α,17α-isopropylidenedioxy-3-oxoandrost-4-ene 17β-thiocarboxylate;
ethyl 4,6α-difluoro-11β-hydroxy-16α,17α-isopropylidenedioxy-3-oxoandrost-4-ene 17β-thiocarboxylate;
phenyl 4-chloro-6α-fluoro-11β-hydroxy-16α,17α-isopropylidenedioxy-3-oxoandrost-4-ene 17β-thiocarboxylate;
benzyl 4-chloro-11β-hydroxy-16α,17α-isopropylidenedioxy-3-oxoandrost-4-ene 17β-thiocarboxylate;
methyl 4-bromo-6α-fluoro-11β-hydroxy-16α,17α-isopropylidenedioxy-3-oxoandrost-4-ene 17β-thiocarboxylate; and the like.

Example 7

This example sets forth an alternative method for preparing the compounds of this invention according to Reaction Sequence B through intermediates (13), (14), (15) and (17).

A mixture of 600 mg of 6α,9α-difluoro-11β-hydroxy-16α-methyl-3-oxo-17α-propionyloxy-androsta-1,4-diene 17β-carboxylic acid, 8 ml of THF and 0.2 ml of TEA are placed in a suitable reaction vessel and stirred at room temperature under a nitrogen blanket. A mixture of 0.24 g of DCP in 8 ml of THF is added over thirteen minutes. Stirring is continued for about six hours at which time 0.04 ml TEA is added, followed by 0.05 g DCP in 3 ml THF. The mixture is stirred for about 18 hours and the resulting precipitate is filtered off, then washed with 10 ml of THF. 2.05 Ml of a solution made from 20 ml dimethylformamide (DMF), 0.758 g 57% sodium hydride and 0.86 g methyl sulfide (DMS) is added to the reaction mixture. The resulting mixture is stirred for about 5$^1/_2$ hours when 1 additional ml of the DMS solution is added and stirring is continued for another hour and one half. The resulting mixture is poured into 200 ml of ethyl acetate, washed twice with 200 ml of water, washed with brine once, then dried overnight in a refrigerator over sodium sulfate, filtered to remove any solids, and finally the solvent is removed on a rotary evaporator. This results in 0,235 g crude material which is then recrystallized from an acetone/hexane mixture to yield 54.3 mg of methyl 6α,9α-difluoro-11β-hydroxy-16α-methyl-3-oxo-17α-propionyloxyandrosta-1,4-diene 17β-thiocarboxylate, m. p. 305—308° C.

Six g of a compound prepared in this manner are mixed with 72 ml of trimethylorthoformate and 24 ml of anhydrous methanol along with 0.3 ml fuming sulfuric acid. The mixture is heated at 50° C for about 30 minutes at which time TLC using a 35% ethyl acetate-65% hexane system shows the reaction to be complete. Ten ml of triethylamine is added and the solvents are eliminated by vacuum evaporation. The residue is dissolved in 30 ml of methanol and slowly diluted with water whereupon crystals form. When a total volume of 2 liters is obtained, the crystalline precipitate is collected by filtration.

The resulting wet cake is dissolved in 300 ml of acetone with 30 ml of water and a stream of $ClO_3F$ is slowly passed through the resulting solution for 10 minutes. TLC analysis of the reaction mixture using 35% ethyl acetate-65% hexane shows the reaction to be complete. The mixture is diluted with 100 ml of water, and the acetone is eliminated under reduced pressure. The remaining liquid mixture is diluted to 1 liter with water while a semi-crystalline precipitate forms. The precipitate is collected by filtration, dissolved in DCM the water layer separated and the DCM solution dried over anhydrous sodium sulfate and filtered. The resulting filtrate is percolated through 70 g of silica while eluting with 100% DCM. The 100% DCM is collected, then the combined eluates are concentrated to dryness and the resulting residue is dissolved in about 10 ml of methanol and diluted slowly with water up to 2 liters while crystallization takes place. The resulting precipitate is collected by filtration, washed with water and dried on a steam bath to give 2.55 g of methyl 4α,6,9α-trifluoro-11β-hydroxy-16α-methyl-3-oxo-17α-propionyloxyandrosta-1,5-diene 17β-thiocarboxylate, m.p. 198—199.5° C, $[\alpha]_D$ 39° (chloroform).

One g of this compound is mixed with 25 ml of methanol in 200 mg of anhydrous potassium carbonate in a suitable reaction vessel under a nitrogen atmosphere at room temperature under stirring for a period of 2 hours, at which time TLC analysis shows the reaction to be complete. One ml acetic acid is added and the methanol is eliminated under reduced pressure to form a small liquid volume which is then diluted with water up to 0.5 liters. The resulting crystalline material is collected by filtration and dried to give 960 mg of a material which is then recrystallized from methylene chloride/methanol. The solvents are eliminated to give 560 mg of methyl 4,6α,9α-trifluoro-11β-hydroxy-16α-methyl-3-oxo-17α-propionyloxyandrosta-1,4-diene 17β-thiocarboxylate, m.p. 287.5° —290° C.

Similarly, by following the above procedure set forth in this example but substituting other alkyl,

benzyl or phenyl sulfides for methyl sulfide in the first step, other alkyl, benzyl or phenyl compounds of this invention are prepared.

## Example 8

By following in principle the procedures set forth in Examples 1, 3 and 5 but substituting the corresponding 16β-methyl steroid starting material for the 16α-methyl steroid starting material, the corresponding 16β-methyl steroids of this invention are obtained such as

4,6α,9α-trifluoro-11β-hydroxy-16β-methyl-3-oxo-17α-propionyloxyandrosta-1,4-diene 17β-thiocarboxylic acid;
methyl 4,6α-difluoro-11β-hydroxy-16β-methyl-3-oxo-17α-propionyloxyandrosta-1,4-diene 17β-thiocarboxylate;
methyl 4-fluoro-11β-hydroxy-16β-methyl-3-oxo-17α-propionyloxyandrosta-1,4-diene 17β-thiocarboxylate;
methyl 17α-acetoxy-4,9α-difluoro-11β-hydroxy-16β-methyl-3-oxoandrosta-1,4-diene 17β-thiocarboxylate, m.p. 265–266° C;
methyl 17α-caproyloxy-4,9α-difluoro-11β-hydroxy-16β-methyl-3-oxoandrosta-1,4-diene 17β-thiocarboxylate, m.p. 164–166° C;
and other 17α-alkanoyloxy derivates along with other alkyl, benzyl and phenyl 17β-thiocarboxylates.

## Example 9

By following in principle the procedures set forth in Examples 1, 3 and 5 but substituting the corresponding 16-unsubstituted steroid starting material for the 16α-methyl steroid starting material, the corresponding unsubstituted steroids of this invention are obtained, such as 4,6α,9α-trifluoro-11β-hydroxy-3-oxo-17α-propionyloxyandrosta-1,4-diene 17β-thiocarboxylic acid and the corresponding 17α-alkanoyloxy derivatives along with the corresponding alkyl, benzyl and phenyl 17β-thiocarboxylates.

## Example 10

This example sets forth a process for hydrogenating the androsta-1,4-dienes to androst-4-enes of this invention.

A solution of 25 mg of tris-(triphenylphosphine) chlororhodium in 6 ml of benzene and 15 ml of ethanol is stirred under hydrogen for 60 minutes. Methyl 4,6α,9α-trifluoro-11β-hydroxy-16α-methyl-3-oxo-17α-propionyloxyandrosta-1,4-diene-17β-thiocarboxylate (250 mg) is added and the resulting solution is stirred under hydrogen at room temperature at atmospheric pressure. After hydrogen uptake is complete, the solution is evaporated to dryness and the residue taken up in a mixture of petroleum ether and methylene chloride. The pure product is isolated by column chromatography on silica gel to give methyl 4,6α,9α-trifluoro-11β-hydroxy-16α-methyl-3-oxo-17α-propionyloxyandrost-4-ene-17β-thiocarboxylate; m.p. 248–249° C.

Similarly, by substituting other androsta-1,4-dienes of this invention made according to Examples 1–3 and 5–8 for the compound of formula (I), other corresponding androst-4-enes are prepared such as n-hexyl 17α-acetoxy-4-chloro-6α-fluoro-11β-hydroxy 17α-methyl-3-oxoandrosta-4-ene 17β-thiocarboxylate, and the like.

## Example 11

This example sets forth a process for preparing an 11-keto compound of this invention by oxidizing any of the 11beta-hydroxy steroids set forth in Preparations I–VI.

One g of 4,6α,9α-trifluoro-11β-hydroxy-16α-methyl-3-oxo-17α-propionyloxyandrost-1,4-diene 17β carboxylic acid is dissolved in 50 ml of acetone and treated at room temperature with Jone's reagent (chromic anhydride in dilute sulfuric acid) dropwise until TLC indicates the absence of starting material. The mixture is treated with five drops of isopropyl alcohol to destroy any excess of reagent, then diluted with 50 ml of water and the mixture concentrated under vacuum under reduced pressure to give a crystalline material, namely 4,6α,9α-trifluoro-16α-methyl-3,11,-dioxo-17α-propionyloxyandrost-1,4-diene 17β-carboxylic acid.

The resulting compound is then reacted according to procedures of Examples 1–6 to obtain an 11-keto steroid of this invention.

Example 12

A mixture of 0.5 g of methyl 4,6α-difluoro-11β-hydroxy-3-oxo-17α-propionyloxyandrost-4-ene 17β-thiocarboxylate, 10 ml of dioxane and 0.35 g of 2,3-dichloro-5,6-dicyano-1,4-benzoquinone is refluxed for 10 hours. The mixture is then cooled, filtered and evaporated to dryness. The residue is dissolved in acetone and this solution is then filtered through 10 g of alumina and concentrated to yield methyl 4,6α-difluoro-11β-hydroxy-3-oxo-17α-propionyloxyandrosta-1,4-diene 17β-thiocarboxylate, which is further purified by recrystallization from acetone : hexane.

Other androsta-1,4-diene 17β-carboxylates of this invention are prepared by following in principle the above procedure but substituting other appropriate androst-4-ene 17β thiocarboxylates for the above-named starting material.

Example 13 — $LD_{50}$

Six Swiss-Webster mice (Simonsen) each weighing about 25 grams, were injected subcutaneously with a solution of methyl 4,6α,9α-trifluoro-11β-hydroxy-16α-methyl-3-oxo-17α-n-propionyloxyandrosta-1,4-diene 17β-thiocarboxylate in carbomethoxycellulose having a concentration of 10 ml/kg. The dosage was 25 mg/kg or about .625 mg/mouse. The mice were observed daily for mortality for 21 days. No mice died. The $LD_{50}$ is, therefore, more than 25 mg/kg.

Example 14 — Biological Activity
Anti-inflammatory Activity

This example sets forth the results of the topical anti-inflammatory activity of a compound (4,6α,9α-trifluoro-11β-hydroxy-16α-methyl-17α-propionyloxyandrosta-1,4-diene 17β-thiocarboxylate) of this invention as compared to fluocinolone acetonide (FA). The topical anti-inflammatory activity potential for each compound was assayed using a modified Stoughton/McKenzie vaso-constriction assay in humans as described by V.A. Place, et al in Arch. Dermat. 101, 531—537 (1970).

Eight normal adult human subjects were treated on four sites on each forearm by topical administration with alcoholic solutions containing $1 \times 10^{-5}$ and $1 \times 10^{-6}$ g/ml of each of the compounds to provide 64 total test sites for each compound in a series (32 for each concentration). Areas of the subjects' forearms were outlined by a rubber stamp grid coated with silicone grease, and 10 microliters of each solution were applied per 7×7 mm. square site. After the preparations dried, the areas on each forearm are covered with Saran$^R$ wrap and the margins sealed with tape. The occlusive wrap is removed after 18 hours. Twenty-four hours after application, the presence or absence of vasoconstriction is noted by visual examination by two independent observers, and expressed as the number of sites responding (vasoconstriction) and is calculated as a percentage of the total number of sites. Also, the intensity of the vasoconstriction is cored on a 0, 1, 2 scale, 2 being the most intense reaction. Both scores are used in constructing dose-response graphs according to methods set forth in an article by Place, et al, infra.

Thymolytic Activity

The same compound was tested for thymolytic activity versus a hydrocortisone standard and each was prepared in three or more concentrations by suspension in a sodium carboxymethyl-cellulose vehicle. Animals received the test materials by subcutaneous injection of 0.5 ml of the suspension on each of three successive days. Four hours following the final injection, the rats were sacrificed and the thymus gland of each animal removed and weighed. These weights are then employed to establish dose-response graphs by a method known in the art. The compound tested showed no activity in the dose range of hydrocortisone (the standard) and therefore is labelled inactive (less than 2 × hydrocortisone in potency).

The results of these tests indicate that the compound tested has good topical anti-inflammatory activity (0.15 times that of FA) while exhibiting little thymolytic activity (less than 2 × hydrocortisone). Thus, the compound tested offers a good therapeutic advantage in that the desired activity is obtained without accompanying adverse systemic side effects.

Other compounds of this invention offer a similar therapeutic advantage.

## Example 15 — Formulation

In this example a formulation is prepared of the following composition

| | % w/w |
|---|---|
| Methyl 4,6α,9α-trifluoro-11β-hydroxy-16α-methyl-3-oxo-17α-propionyloxyandrosta-1,4-diene 17β-thiocarboxylate | 0.025 |
| Stearyl Alcohol | 30.0 |
| PEG 6000 (Trade Mark) | 5.0 |
| 1,2,6-Hexanetriol | 2.5 |
| Citric Acid Anhydrous, USP | 0.02 |
| Propylene Glycol, USP, q.s. | 100.0 |

The steroid is dissolved in 624.8 grams of propylene glycol at 90 – 95° C. The latter is then mixed with the other ingredients at 80 – 85° C to give the desired formulation.

## Example 16

This example sets forth an alternative method for preparing the 16β-methyl steroids of this invention.

A. Ten (10) g of 6α-fluoro-16β-methyl-17α,21-diacetoxypregna-1,4,9(11)-triene-3,20-dione in 110 mls of dioxane (A.R.) plus 2.2 mls of a solution of 4.4 mls 70% $HClO_4$ in 200 mls of water is treated with 4 g of dibromantoin in the dark at room temperature for one hour when TLC in 50% ethyl acetate/50% Hexane shows the reaction to be complete. The reaction mixture is diluted with 2 liters of water, stirred for 10 minutes and the crystalline precipitate collected by filtration, washed with water, and air dried to give 11.4 g of 6α-fluoro-9α-bromo-11β-hydroxy-16β-methyl-17α,21-diacetoxypregna 1,4-diene-3,20-dione.

This bromohydrin (19.1 g) is mixed with 286 ml of methyl orthoformate, 96 ml of anhydrous methanol and 1.9 ml of fuming sulfuric acid and heated on a water bath at 80 – 85° for 15 minutes. The mixture is treated with 15 ml of pyridine and poured into 300 ml of water, separated and washed three times with water, dried over anhydrous sodium sulfate, filtered and concentrated under high vacuum to a foam which is cooled in crushed dry ice for 16 hours to give the 11β-orthoester of 3-methoxy-6α-fluoro-9α-bromo-16β-methyl-17α,21-diacetoxypregna-1,3,5(6)-triene-20-one.

The orthoester so obtained is dissolved in 300 ml of a mixture of 80% THF/20% water and treated at room temperature with a slow stream of perchloryl fluoride until no more starting material was detected by TLC analysis. The mixture is diluted with water and the organic solvent eliminated under reduced pressure (high vacuum) at 80 – 85° C. The mixture is diluted with water up to two 1 and kept in the refrigerator for 20 hours. The resulting precipitate is filtered and air dried.

One (1) g of crude reaction mixture is dissolved in about 20 mls of methylene dichloride (MDC) and filtered through a 10 g column of silica with 100% MDC. The column was eluted with 1.2 liters of MDC, then with a mixture of 2% ethyl acetate/98% MDC. The homogeneous fractions containing small amounts of negative and positive polar impurities are concentrated to dryness under a high vacuum. NMR analysis of the negative polar product eluted indicates that the product is 4,6α-difluoro-9α-bromo-11β-hydroxy-16β-methyl-17α,21-diacetoxypregna-1,5(6)-diene-3,20-dione.

The resulting product is mixed with tin tributylhydride in tetrahydrofuran at room temperature to eliminate the 9α-bromine and form 4,6α-difluoro-11β-hydroxy-16β-methyl-17α,21-diacetoxypregna-1,5(6)-3,20-dione (The reaction may be accelerated by adding a small amount of a free radical and refluxing).

The resulting product is stirred with methanol containing anhydrous potassium carbonate under nitrogen at atmospheric pressure and ambient temperature until TLC shows the reaction is complete. The reaction mixture is diluted with methanol and glacial acetic acid and concentrated under reduced pressure to a small volume. The crystalline precipitate which forms is collected by filtration and washed with methanol and water to give 4,6α-difluoro-11β-hydroxy-16β-methyl-17α,21-dihydroxypregna-1,4-diene-3,20-dione.

The resulting product is reacted with aqueous periodic acid ($H_5IO_6$) in methanol at room temperature until the reaction is complete as judged by TLC. The methanol is removed by evaporation, water is added and the resulting precipitate removed by filtration and purified by crystallization to give 4,6α-difluoro-11β,17α-dihydroxy-16β-methyl-3-oxoandrosta-1,4-diene 17β-carboxylic acid.

This product is then reacted with propionic anhydride and pyridine according to the process set forth in Preparation I, Part A to give the corresponding 17α-propionyloxy derivative which, in turn, is reacted in principle according to the process of Example 1, Part A to give methyl 4,6α-difluoro-11β-hydroxy-16β-methyl-3-oxo-17-propionyloxyandrosta-1,4-diene 17β-thiocarboxylate.

B. The final compound resulting from Part A of this example is further elaborated into other compounds of this invention by first forming the corresponding androsta-1,4,9(11)-triene. This is accomplished by any means known in the art such as dissolving methyl 4,6α-difluoro-11β-hydroxy-16β-methyl-3-oxo-17-propionyloxyandrosta-1,4-diene 17β-thiocarboxylate in DMF, adding methylsulfonyl chloride and pyridine and heating at about 80° C until the reaction is complete. The product is extracted with an organic solvent such as ethyl acetate, washed with water, dried over sodium sulfate and evaporated to yield methyl 4,6α-difluoro-16β-methyl-3-oxo-17α-propionyloxyandrosta-1,4,9(11)-triene 17β-thiocarboxylate. This, in turn, is chlorinated using chlorine in carbon tetrachloride according to methods known in the art to give methyl 4,6α-difluoro-9α,11β-dichloro-16β-methyl-3-oxo-17α-propionyloxyandrosta-1,4-diene 17β-thiocarboxylate.

C. By reacting 4,6α-difluoro-11β,17α-dihydroxy-16β-methyl-3-oxoandrosta-1,4-diene 17β-carboxylic acid with acetic anhydride, propionic anhydride or butyric anhydride according to the process set forth in Preparation I, Part A and other alkyl sulfides such as ethyl sulfide or isopropyl sulfide as set forth in Example 1, Part B other compounds of this invention are prepared such as

ethyl 4,6α-difluoro-11β-hydroxy-16β-methyl-3-oxo-17-propionyloxyandrosta-1,4-diene 17β-thiocarboxylate;
ethyl 17α-acetoxy-4,6α-difluoro-11β-hydroxy-16β-methyl-3-oxoandrosta-1,4-diene 17β-thiocarboxylate;
isopropyl 17α-butyryloxy-4,6α-difluoro-11β-hydroxy-16β-methyl-3-oxoandrosta-1,4-diene 17β-thiocarboxylate;
and the like.

## Claims

1. A compound chosen from those represented by the formula

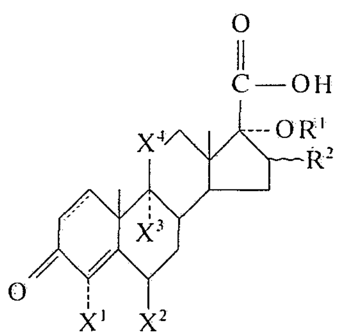

wherein

$X^1$ is fluoro, chloro or bromo;
$X^2$ is fluoro, chloro or hydrogen;
$X^3$ is fluoro, chloro, bromo or hydrogen;
$X^4$ is =C=O or

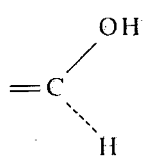

or may also be

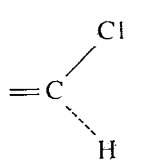

when $X^3$ is chloro;

R is alkyl of 1 to 6 carbon atoms, or phenyl or benzyl optionally substituted with one substituent on the phenyl ring chosen from the group consisting of alkyl of 1 to 4 carbon atoms, alkoxy of 1 to 4 carbon atoms and halo;
$R^1$ is hydrogen or alkanoyl of 2 to 6 carbon atoms when $R^2$ is hydrogen, $\alpha$-methyl or $\beta$-methyl, or $OR^1$ and $R^2$ together are isopropylidenedioxy; and the solid and broken lines between C-1 and C-2 represent a double or a single bond.

2. A compound of Claim 1 wherein

R is alkyl of one to six carbon atoms;
$R^1$ is alkanoyl of two to six carbon atoms when $R^2$ is $\alpha$-methyl or $\beta$-methyl or $OR^1$ and $R^2$ together are $16\alpha,17\alpha$-isopropylidenedioxy;
$X^1$ is fluoro or chloro;
$X^2$ is fluoro or hydrogen;
$X^3$ is fluoro, chloro or hydrogen; and
$X^4$ is

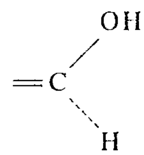

or may also be

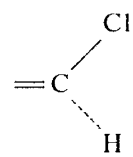

when $X^3$ is chloro.

3. A compound of Claim 2 wherein

$R^2$ is $\alpha$-methyl or $\beta$-methyl;
$X^1$ is fluoro;
$X^2$ is hydrogen or fluoro;
$X^3$ is hydrogen or fluoro;
X is

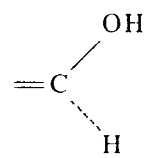

and
there is a double bond between C-1 and C-2.

4. A compound of Claim 3 wherein R is methyl; $R^1$ is alkanoyl of 2 or 3 carbon atoms; and $X^1$, $X^2$ and $X^3$ are all fluoro.

5. A compound of Claim 1 wherein R is alkyl of one to six carbon atoms, $OR^1$ and $R^2$ together are isopropylidenedioxy, $X^4$ is

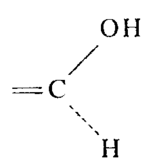

$X^1$ is fluoro and $X^2$ and $X^3$ are each independently fluoro or hydrogen.

6. An anti-inflammatory pharmaceutical composition which comprises a therapeutically effective amount of a compound of Claim 1 in combination with at least one suitabie pharmaceutical excipient.

7. A compound of claim 1 for use in treating an inflamed condition in a mammal.

8. A process for preparing a compound of Claim 1 which comprises treating a compound of the formula

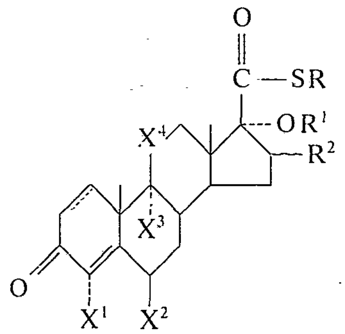

wherein $X^1$, $X^2$, $X^3$, $X^4$, $R^1$ and $R^2$ are as defined in Claim 1 or a corresponding reactive derivative thereof with a suitable base salt of a compound represented by RSH wherein R is as defined in Claim 1.

9. A process for preparing a compound of Claim 1 wherein $R^1$ is alkanoyl of two to six carbon atoms and $X^1$, $X^2$, $X^3$, $X^4$, R, $R^2$ and the broken line between C-1 and C-2 are as defined in Claim 1, which process comprises esterifying the corresponding 17α-hydroxy compound.

10. A process for preparing a compound of Claim 1, which process comprises contacting a compound represented by the formula

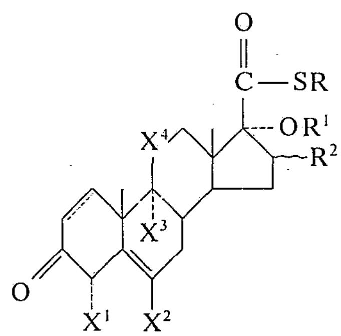

wherein R, $R^1$, $R^2$, $X^1$, $X^2$, $X^3$, $X^4$ and the broken line between C-1 and C-2 are as defined in Claim 1 with a base, in a suitable oxygenated hydrocarbon solvent, in an inert atmosphere.

**Patentansprüche**

1. Eine Verbindung, ausgewählt aus solchen gemäß der Formel:

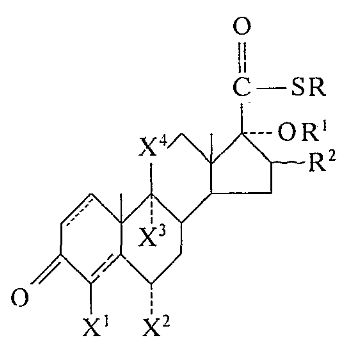

in welcher

$X^1$ Fluor, Chlor oder Brom ist;
$X^2$ Fluor, Chlor oder Wasserstoff ist;
$X^3$ Fluor, Chlor, Brom oder Wasserstoff ist;

$X^4$ =C=O oder

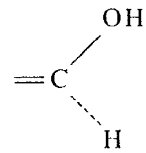

ist; oder auch für

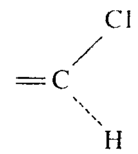

stehen kann, wenn $X^3$ Chlor ist;

R eine Alkylgruppe mit 1 bis 6 Kohlenstoffatomen oder eine Phenyl- oder Benzylgruppe ist, die gegebenenfalls auf dem Phenylring mit einem Substituenten aus der Gruppe von Alkylgruppen mit 1 bis 4 Kohlenstoffatomen, Alkoxygruppen mit 1 bis 4 Kohlenstoffatomen und Halogen substituiert ist;

$R^1$ Wasserstoff oder eine Alkanoylgruppe mit 2 bis 6 Kohlenstoffatomen ist, wenn $R^2$ Wasserstoff, $\alpha$-Methyl oder $\beta$-Methyl ist; oder $OR^1$ und $R^2$ stehen zusammen für Isopropylidendioxy; und

die durchgezogenen und unterbrochenen Linien zwischen C-1 und C-2 eine Doppel- oder Einfachbindung bedeuten.

2. Verbindung nach Anspruch 1, in welcher

R eine Alkylgruppe mit 1 bis 6 Kohlenstoffatomen ist;

$R^1$ eine Alkanoylgruppe mit 2 bis 6 Kohlenstoffatomen ist, wenn $R^2$ für $\alpha$-Methyl oder $\beta$-Methyl steht; oder $OR^1$ und $R^2$ stehen zusammen für $16\alpha,17\alpha$-Isopropylidendioxy;

$X^1$ Fluor oder Chlor ist;

$X^2$ Fluor oder Wasserstoff ist;

$X^3$ Fluor, Chlor oder Wasserstoff ist; und

$X^4$

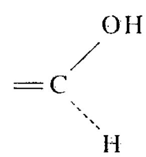

ist; oder auch

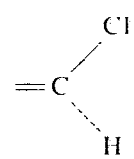

sein kann, wenn $X^3$ Chlor ist.

3. Verbindung nach Anspruch 2, in welcher

$R^2$ $\alpha$-Methyl oder $\beta$-Methyl ist;

$X^1$ Fluor ist;

$X^2$ Wasserstoff oder Fluor ist;

$X^3$ Wasserstoff oder Fluor ist;

X

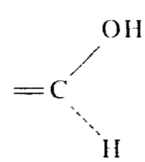

ist;

und eine Doppelbindung zwischen C-1 und C-2 vorliegt.

4. Verbindung nach Anspruch 3, in welcher R Methyl ist; $R^1$ eine Alkanoylgruppe mit 2 oder 3 Kohlenstoffatomen ist, und $X^1$, $X^2$ und $X^3$ jeweils für Fluor stehen.

5. Verbindung nach Anspruch 1, in welcher R für eine Alkylgruppe mit 1 bis 6 Kohlenstoffatomen steht; $OR^1$ und $R^2$ zusammen Isopropylidendioxy bedeuten; $X^4$

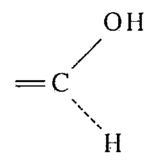

ist; $X^1$ Fluor ist und $X^2$ und $X^3$ jeweils unabhängig Fluor oder Wasserstoff bedeuten.

6. Entzündungshemmendes, pharmazeutisches Präparat, umfassend eine therapeutisch wirksame Menge einer Verbindung gemäß Anspruch 1 in Kombination mit mindestens einem geeigneten, pharmazeutischen Träger.

7. Eine Verbindung gemäß Anspruch 1, zur Verwendung bei der Behandlung einer entzündlichen Erkrankung bei Mensch und Säugetier.

8. Verfahren zur Herstellung einer Verbindung gemäß Anspruch 1, dadurch gekennzeichnet, daß man eine Verbindung der Formel:

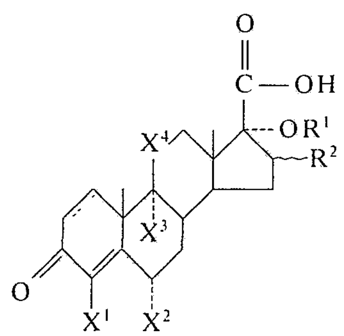

in welcher $X^1$, $X^2$, $X^3$, $X^4$, $R^1$ und $R^2$ wie in Anspruch 1 definiert sind, oder ein entsprechendes, reaktionsfähiges Derivat derselben mit einem geeigneten basischen Salz einer Verbindung der Formel RSH, in welcher R wie in Anspruch 1 definiert ist, behandelt.

9. Verfahren zur Herstellung einer Verbindung gemäß Anspruch 1, in welcher $R^1$ eine Alkanoylgruppe mit 2 bis 6 Kohlenstoffatomen ist und $X^1$, $X^2$, $X^3$, $X^4$, R, $R^2$ und die unterbrochene Linie zwischen C-1 und C-2 wie in Anspruch 1 definiert sind, dadurch gekennzeichnet, daß man die entsprechende 17$\alpha$-Hydroxyverbindung verestert.

10. Verfahren zur Herstellung einer Verbindung gemäß Anspruch 1, dadurch gekennzeichnet, daß man eine Verbindung der Formel

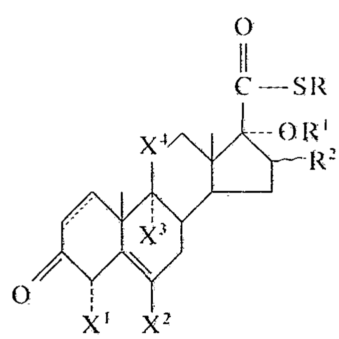

in welcher R, $R^1$, $R^2$, $X^1$, $X^2$, $X^3$, $X^4$ und die unterbrochene Linie zwischen C-1 und C-2 wie in Anspruch 1 definiert sind, mit einer Base in einem geeigneten, sauerstoffhaltigen Kohlenwasserstofflösungsmittel in einer inerten Atmosphäre in Berührung bringt.

**Revendications**

1. Un composé choisi parmi ceux qui sont représentés par la formule:

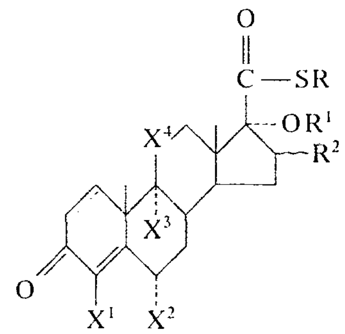

dans laquelle

X¹ est un radical fluoro, chloro ou bromo;
X² est un radical fluoro ou chloro ou de l'hydrogène;
X³ est un radical fluoro, chloro ou bromo ou de l'hydrogène;
X⁴ est un groupe =C=O ou

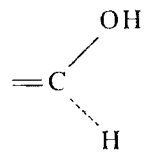

ou peut aussi être un groupe

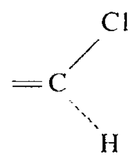

lorsque X³ est un radical chloro;
R est un radical alkyle ayant 1 à 6 atomes de carbone ou phényle ou benzyle éventuellement substitué avec un substituant sur le noyau phényle, choisi dans le groupe comprenant des radicaux alkyle ayant 1 à 4 atomes de carbone, des radicaux alkoxy ayant 1 à 4 atomes de carbone et des radicaux halogéno;
R¹ est de l'hydrogène ou un groupe alcanoyle ayant 2 à 6 atomes de carbone lorsque R² est de l'hydrogène, un groupe $\alpha$-méthyle ou un groupe $\beta$-méthyle, ou bien OR¹ et
R² forment ensemble un groupe isopropylidènedioxy;
et les liaisons en trait plein et en trait interrompu entre les atomes C-1 et C-2 représentent une liaison double ou une liaison simple.

2. Composé suivant la revendication 1, caractérisé en ce que:

R est un groupe alkyle ayant 1 à 6 atomes de carbone;
R¹ est un groupe alcanoyle ayant 2 à 6 atomes de carbone lorsque R² est un groupe $\alpha$-méthyle ou $\beta$-méthyle ou bien OR¹ et R² forment ensemble un groupe 16$\alpha$,17$\alpha$-isopropylidènedioxy,
X¹ est un radical fluoro ou chloro,
X² est un radical fluoro ou de l'hydrogène;
X³ est un radical fluoro ou chloro ou de l'hydrogène; et
X⁴ est un groupe

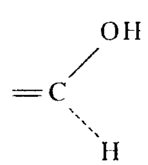

ou peut aussi être un groupe

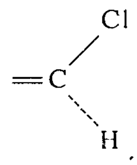

lorsque $X^3$ est un radical chloro.

3. Composé suivant la revendication 2, caractérisé en ce que $R^2$ est un groupe $\alpha$-méthyle ou $\beta$-méthyle;

$X^1$ est un radical fluoro;
$X^2$ est de l'hydrogène ou un radical fluoro;
$X^3$ est de l'hydrogène ou un radical fluoro;
X est un groupe

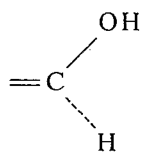

et une double liaison existe entre les atomes C-1 et C-2.

4. Composé suivant la revendication 3, caractérisé en ce que R est un groupe méthyle; $R^1$ est un groupe alcanoyle ayant 2 ou 3 atomes de carbone et $X^1$, $X^2$ et $X^3$ sont tous des radicaux fluoro.

5. Composé suivant la revendication 1, caractérisé en ce que R est un groupe alkyle ayant 1 à 6 atomes de carbone, $OR^1$ et $R^2$ forment ensemble un groupe isopropylidènedioxy; $X^4$ est un groupe

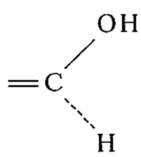

$X^1$ est un radical fluoro et $X^2$ et $X^3$ sont chacun, indépendamment, un radical fluoro ou un atome d'hydrogène.

6. Composition pharmaceutique anti-inflammatoire, caractérisée en ce qu'elle comprend une quantité thérapeutiquement efficace d'un composé suivant la revendication 1 en association avec au moins un excipient pharmaceutique convenable.

7. Composé suivant la revendication 1, destiné à être utilisé dans le traitement d'un état d'inflammation chez un mammifère.

8. Procédé de préparation d'un composé suivant la revendication 1, caractérisé en ce qu'il consiste à traiter un composé de formule

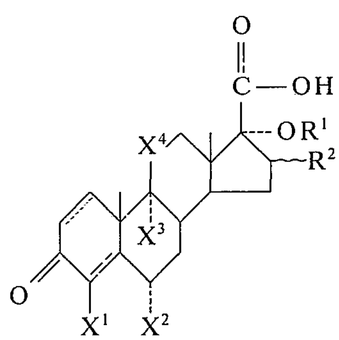

dans laquelle $X^1$, $X^2$, $X^3$, $X^4$, $R^1$ et $R^2$ ont les définitions données dans la revendication 1, ou un dérivé réactif correspondant de ce composé avec un sel basique convenable d'un composé représenté par la formule RSH dans laquelle R a la définition donnée dans la revendication 1.

9. Procédé de préparation d'un composé suivant la revendication 1, dans laquelle $R^1$ est un groupe alcanoyle ayant 2 à 6 atomes de carbone et $X^1$, $X^2$, $X^3$, $X^4$, R, $R^2$ et la liaison interrompue entre les

atomes C-1 et C-2 ont les définitions données dans la revendication 1, procédé caractérisé en ce qu'il consiste à estérifier le composé 17α-hydroxy correspondant.

10. Procédé de préparation d'un composé suivant la revendication 1, caractérisé en ce qu'il consiste à faire entrer un composé de formule:

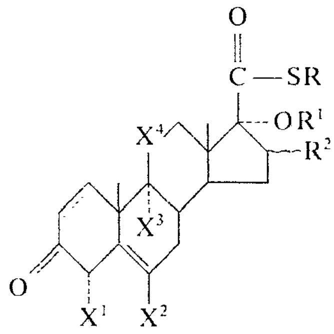

dans laquelle R, $R^1$, $R^2$, $X^1$, $X^2$, $X^3$, $X^4$ et la liaison en traits interrompus entre les atomes C-1 et C-2 ont les définitions données dans la revendication 1, en contact avec une base dans un solvant hydrocarboné oxygéné convenable, en atmosphère inerte.